# EUROPEAN PATENT SPECIFICATION

(11) **EP 3 007 719 B1**
(45) Date of publication and mention of the grant of the patent: **17.03.2021**
(21) Application number: 14810402.9
(22) Date of filing: 11.06.2014
(51) Int. Cl.: A61K 38/18, A61P 25/28

(54) **METHODS AND COMPOSITIONS FOR INCREASING NEUROGENESIS AND ANGIOGENESIS**
VERFAHREN UND ZUSAMMENSETZUNGEN ZUR ERHÖHUNG DER NEUROGENESE UND ANGIOGENESE
PROCÉDÉS ET COMPOSITIONS POUR AUGMENTER LA NEUROGENÈSE ET L'ANGIOGENÈSE

(30) Priority: 11.06.2013 US 201361833813 P; 05.05.2014 US 201461988862 P
(43) Date of publication of application: 20.04.2016
(73) Proprietor: President and Fellows of Harvard College, Cambridge, MA 02138 (US); The Brigham and Women's Hospital, Inc., Boston, MA 02115 (US)
(72) Inventor: RUBIN, Lee, L., Wellesley, MA 02481 (US); WAGERS, Amy, J., Cambridge, MA 02138 (US); LEE, Richard, T., Weston, MA 02193 (US); KATSIMPARDI, Lida, 75013 Paris (FR)
(74) Representative: Lock, Graham James
(86) International application number: PCT/US2014/041952
(87) International publication number: WO 2014/201143

(56) References cited:
- WO-A1-2012/135623
- WO-A2-99/24058
- US-A1- 2003 104 977
- US-A1- 2009 215 671
- US-A1- 2011 027 177
- US-A1- 2011 105 395
- Nicholas R F Hannan ET AL: "BMP-11 and Myostatin Support Undifferentiated Growth of Human Embryonic Stem Cells in Feeder-Free Cultures", , 1 January 2009 (2009-01-01), XP55344871, Retrieved from the Internet: URL:http://online.liebertpub.com/doi/pdf/1 0.1089/clo.2009.0024 [retrieved on 2017-02-13]
- OLOV ANDERSSON ET AL: "Growth differentiation factor 11 signals through the transforming growth factor-[beta] receptor ALK5 to regionalize the anterior-posterior axis", EMBO REPORTS, 14 July 2006 (2006-07-14), XP055344906, GB ISSN: 1469-221X, DOI: 10.1038/sj.embor.7400752
- WU H-H ET AL: "Autoregulation of Neurogenesis by GDF-11", NEURON, CAMBRIDGE, MA, US, vol. 37, 23 January 2003 (2003-01-23), pages 197-207, XP003006666,

### SEQUENCE LISTING

<110> President and Fellows of Harvard College The Brigham and Women's Hospital, Inc.
<120> METHODS AND COMPOSITIONS FOR INCREASING NEUROGENESIS AND ANGIOGENESIS
<130> HRVY-024-WO1
<140> PCT/US2014/041952
   <141> 2014-06-11
<150> US 61/833,813
   <151> 2013-06-11
<160> 5
<170> PatentIn version 3.5
<210> 1
   <211> 407
   <212> PRT
   <213> Homo sapiens
<400> 1
   Met Val Leu Ala Ala Pro Leu Leu Leu Gly Phe Leu Leu Leu Ala Leu
   1               5                   10                  15
   Glu Leu Arg Pro Arg Gly Glu Ala Ala Glu Gly Pro Ala Ala Ala Ala
               20                  25                  30
   Ala Ala Ala Ala Ala Ala Ala Ala Ala Gly Val Gly Gly Glu Arg Ser
           35                  40                  45
   Ser Arg Pro Ala Pro Ser Val Ala Pro Glu Pro Asp Gly Cys Pro Val
       50                  55                  60
   Cys Val Trp Arg Gln His Ser Arg Glu Leu Arg Leu Glu Ser Ile Lys
   65                  70                  75                  80
   Ser Gln Ile Leu Ser Lys Leu Arg Leu Lys Glu Ala Pro Asn Ile Ser
                   85                  90                  95
   Arg Glu Val Val Lys Gln Leu Leu Pro Lys Ala Pro Pro Leu Gln Gln
               100                 105                 110
   Ile Leu Asp Leu His Asp Phe Gln Gly Asp Ala Leu Gln Pro Glu Asp
           115                 120                 125
   Phe Leu Glu Glu Asp Glu Tyr His Ala Thr Thr Glu Thr Val Ile Ser
       130                 135                 140
   Met Ala Gln Glu Thr Asp Pro Ala Val Gln Thr Asp Gly Ser Pro Leu
   145                 150                 155                 160
   Cys Cys His Phe His Phe Ser Pro Lys Val Met Phe Thr Lys Val Leu
                   165                 170                 175
   Lys Ala Gln Leu Trp Val Tyr Leu Arg Pro Val Pro Arg Pro Ala Thr
               180                 185                 190
   Val Tyr Leu Gln Ile Leu Arg Leu Lys Pro Leu Thr Gly Glu Gly Thr
           195                 200                 205
   Ala Gly Gly Gly Gly Gly Gly Arg Arg His Ile Arg Ile Arg Ser Leu
       210                 215                 220
   Lys Ile Glu Leu His Ser Arg Ser Gly His Trp Gln Ser Ile Asp Phe
   225                 230                 235                 240
   Lys Gln Val Leu His Ser Trp Phe Arg Gln Pro Gln Ser Asn Trp Gly
                   245                 250                 255
   Ile Glu Ile Asn Ala Phe Asp Pro Ser Gly Thr Asp Leu Ala Val Thr
               260                 265                 270
   Ser Leu Gly Pro Gly Ala Glu Gly Leu His Pro Phe Met Glu Leu Arg
           275                 280                 285
   Val Leu Glu Asn Thr Lys Arg Ser Arg Arg Asn Leu Gly Leu Asp Cys
       290                 295                 300
   Asp Glu His Ser Ser Glu Ser Arg Cys Cys Arg Tyr Pro Leu Thr Val
   305                 310                 315                 320
   Asp Phe Glu Ala Phe Gly Trp Asp Trp Ile Ile Ala Pro Lys Arg Tyr
                   325                 330                 335
   Lys Ala Asn Tyr Cys Ser Gly Gln Cys Glu Tyr Met Phe Met Gln Lys
               340                 345                 350
   Tyr Pro His Thr His Leu Val Gln Gln Ala Asn Pro Arg Gly Ser Ala
           355                 360                 365
   Gly Pro Cys Cys Thr Pro Thr Lys Met Ser Pro Ile Asn Met Leu Tyr
       370                 375                 380
   Phe Asn Asp Lys Gln Gln Ile Ile Tyr Gly Lys Ile Pro Gly Met Val
   385                 390                 395                 400
   Val Asp Arg Cys Gly Cys Ser
   405
<210> 2
   <211> 383
   <212> PRT
   <213> Homo sapiens
<400> 2
   Ala Glu Gly Pro Ala Ala Ala Ala Ala Ala Ala Ala Ala Ala Ala Ala
   1               5                   10                  15
   Ala Gly Val Gly Gly Glu Arg Ser Ser Arg Pro Ala Pro Ser Val Ala
               20                  25                  30
   Pro Glu Pro Asp Gly Cys Pro Val Cys Val Trp Arg Gln His Ser Arg
           35                  40                  45
   Glu Leu Arg Leu Glu Ser Ile Lys Ser Gln Ile Leu Ser Lys Leu Arg
       50                  55                  60
   Leu Lys Glu Ala Pro Asn Ile Ser Arg Glu Val Val Lys Gln Leu Leu
   65                  70                  75                  80
   Pro Lys Ala Pro Pro Leu Gln Gln Ile Leu Asp Leu His Asp Phe Gln
                   85                  90                  95
   Gly Asp Ala Leu Gln Pro Glu Asp Phe Leu Glu Glu Asp Glu Tyr His
               100                 105                 110
   Ala Thr Thr Glu Thr Val Ile Ser Met Ala Gln Glu Thr Asp Pro Ala
           115                 120                 125
   Val Gln Thr Asp Gly Ser Pro Leu Cys Cys His Phe His Phe Ser Pro
       130                 135                 140
   Lys Val Met Phe Thr Lys Val Leu Lys Ala Gln Leu Trp Val Tyr Leu
   145                 150                 155                 160
   Arg Pro Val Pro Arg Pro Ala Thr Val Tyr Leu Gln Ile Leu Arg Leu
                   165                 170                 175
   Lys Pro Leu Thr Gly Glu Gly Thr Ala Gly Gly Gly Gly Gly Gly Arg
               180                 185                 190
   Arg His Ile Arg Ile Arg Ser Leu Lys Ile Glu Leu His Ser Arg Ser
           195                 200                 205
   Gly His Trp Gln Ser Ile Asp Phe Lys Gln Val Leu His Ser Trp Phe
       210                 215                 220
   Arg Gln Pro Gln Ser Asn Trp Gly Ile Glu Ile Asn Ala Phe Asp Pro
   225                 230                 235                 240
   Ser Gly Thr Asp Leu Ala Val Thr Ser Leu Gly Pro Gly Ala Glu Gly
                   245                 250                 255
   Leu His Pro Phe Met Glu Leu Arg Val Leu Glu Asn Thr Lys Arg Ser
               260                 265                 270
   Arg Arg Asn Leu Gly Leu Asp Cys Asp Glu His Ser Ser Glu Ser Arg
           275                 280                 285
   Cys Cys Arg Tyr Pro Leu Thr Val Asp Phe Glu Ala Phe Gly Trp Asp
       290                 295                 300
   Trp Ile Ile Ala Pro Lys Arg Tyr Lys Ala Asn Tyr Cys Ser Gly Gln
   305                 310                 315                 320
   Cys Glu Tyr Met Phe Met Gln Lys Tyr Pro His Thr His Leu Val Gln
                   325                 330                 335
   Gln Ala Asn Pro Arg Gly Ser Ala Gly Pro Cys Cys Thr Pro Thr Lys
               340                 345                 350
   Met Ser Pro Ile Asn Met Leu Tyr Phe Asn Asp Lys Gln Gln Ile Ile
           355                 360                 365
   Tyr Gly Lys Ile Pro Gly Met Val Val Asp Arg Cys Gly Cys Ser
       370                 375                 380
<210> 3
   <211> 109
   <212> PRT
   <213> Homo sapiens
<400> 3
   Asn Leu Gly Leu Asp Cys Asp Glu His Ser Ser Glu Ser Arg Cys Cys
   1               5                   10                  15
   Arg Tyr Pro Leu Thr Val Asp Phe Glu Ala Phe Gly Trp Asp Trp Ile
               20                  25                  30
   Ile Ala Pro Lys Arg Tyr Lys Ala Asn Tyr Cys Ser Gly Gln Cys Glu
           35                  40                  45
   Tyr Met Phe Met Gln Lys Tyr Pro His Thr His Leu Val Gln Gln Ala
       50                  55                  60
   Asn Pro Arg Gly Ser Ala Gly Pro Cys Cys Thr Pro Thr Lys Met Ser
   65                  70                  75                  80
   Pro Ile Asn Met Leu Tyr Phe Asn Asp Lys Gln Gln Ile Ile Tyr Gly
                   85                  90                  95
   Lys Ile Pro Gly Met Val Val Asp Arg Cys Gly Cys Ser
               100                 105
<210> 4
   <211> 274
   <212> PRT
   <213> Homo sapiens
<400> 4
   Ala Glu Gly Pro Ala Ala Ala Ala Ala Ala Ala Ala Ala Ala Ala Ala
   1               5                   10                  15
   Ala Gly Val Gly Gly Glu Arg Ser Ser Arg Pro Ala Pro Ser Val Ala
               20                  25                  30
   Pro Glu Pro Asp Gly Cys Pro Val Cys Val Trp Arg Gln His Ser Arg
           35                  40                  45
   Glu Leu Arg Leu Glu Ser Ile Lys Ser Gln Ile Leu Ser Lys Leu Arg
       50                  55                  60
   Leu Lys Glu Ala Pro Asn Ile Ser Arg Glu Val Val Lys Gln Leu Leu
   65                  70                  75                  80
   Pro Lys Ala Pro Pro Leu Gln Gln Ile Leu Asp Leu His Asp Phe Gln
                   85                  90                  95
   Gly Asp Ala Leu Gln Pro Glu Asp Phe Leu Glu Glu Asp Glu Tyr His
               100                 105                 110
   Ala Thr Thr Glu Thr Val Ile Ser Met Ala Gln Glu Thr Asp Pro Ala
           115                 120                 125
   Val Gln Thr Asp Gly Ser Pro Leu Cys Cys His Phe His Phe Ser Pro
       130                 135                 140
   Lys Val Met Phe Thr Lys Val Leu Lys Ala Gln Leu Trp Val Tyr Leu
   145                 150                 155                 160
   Arg Pro Val Pro Arg Pro Ala Thr Val Tyr Leu Gln Ile Leu Arg Leu
                   165                 170                 175
   Lys Pro Leu Thr Gly Glu Gly Thr Ala Gly Gly Gly Gly Gly Gly Arg
               180                 185                 190
   Arg His Ile Arg Ile Arg Ser Leu Lys Ile Glu Leu His Ser Arg Ser
           195                 200                 205
   Gly His Trp Gln Ser Ile Asp Phe Lys Gln Val Leu His Ser Trp Phe
       210                 215                 220
   Arg Gln Pro Gln Ser Asn Trp Gly Ile Glu Ile Asn Ala Phe Asp Pro
   225                 230                 235                 240
   Ser Gly Thr Asp Leu Ala Val Thr Ser Leu Gly Pro Gly Ala Glu Gly
                   245                 250                 255
   Leu His Pro Phe Met Glu Leu Arg Val Leu Glu Asn Thr Lys Arg Ser
               260                 265                 270
   Arg Arg
<210> 5
   <211> 4260
   <212> DNA
   <213> Homo sapiens
<400> 5
   tccccgcccc ccagtcctcc ctcccctccc ctccagcatg gtgctcgcgg ccccgctgct      60
   gctgggcttc ctgctcctcg ccctggagct gcggccccgg ggggaggcgg ccgagggccc     120
   cgcggcggcg gcggcggcgg cggcggcggc ggcagcggcg ggggtcgggg gggagcgctc     180
   cagccggcca gccccgtccg tggcgcccga gccggacggc tgccccgtgt gcgtttggcg     240
   gcagcacagc cgcgagctgc gcctagagag catcaagtcg cagatcttga gcaaactgcg     300
   gctcaaggag gcgcccaaca tcagccgcga ggtggtgaag cagctgctgc ccaaggcgcc     360
   gccgctgcag cagatcctgg acctacacga cttccagggc gacgcgctgc agcccgagga     420
   cttcctggag gaggacgagt accacgccac caccgagacc gtcattagca tggcccagga     480
   gacggaccca gcagtacaga cagatggcag ccctctctgc tgccattttc acttcagccc     540
   caaggtgatg ttcacaaagg tactgaaggc ccagctgtgg gtgtacctac ggcctgtacc     600
   ccgcccagcc acagtctacc tgcagatctt gcgactaaaa cccctaactg gggaagggac      660
   cgcaggggga gggggcggag gccggcgtca catccgtatc cgctcactga agattgagct     720
   gcactcacgc tcaggccatt ggcagagcat cgacttcaag caagtgctac acagctggtt 780
   ccgccagcca cagagcaact ggggcatcga gatcaacgcc tttgatccca gtggcacaga 840
   cctggctgtc acctccctgg ggccgggagc cgaggggctg catccattca tggagcttcg 900
   agtcctagag aacacaaaac gttcccggcg gaacctgggt ctggactgcg acgagcactc 960
   aagcgagtcc cgctgctgcc gatatcccct cacagtggac tttgaggctt tcggctggga 1020
   ctggatcatc gcacctaagc gctacaaggc caactactgc tccggccagt gcgagtacat 1080
   gttcatgcaa aaatatccgc atacccattt ggtgcagcag gccaatccaa gaggctctgc     1140
   tgggccctgt tgtaccccca ccaagatgtc cccaatcaac atgctctact tcaatgacaa     1200
   gcagcagatt atctacggca agatccctgg catggtggtg gatcgctgtg gctgctctta 1260
   aggtggggga tagaggatgc ctcccccaca gaccctaccc caagacccct agccctgccc 1320
   ccatcccccc aagccctaga gctccctcca ctcttcccgc gaacatcaca ccgttccccg 1380
   accaagccgt gtgcaataca acagagggag gcaggtggga attgagggtg aggggtttgg 1440
   gggaaagggg aagcaggggc atagtcaggg tggggagtgt ttgaagtttg cagatgagaa 1500
   ggtttgacaa aaagacagag agatgtagag acagtgatag agacagagga acaaaaagag 1560
   cagcagtgag aaggcaaaga gagaggcaga agagacagac gaggcagaga caaaacactg 1620
   agaaagagac tgaaatggag taataaatga aagccccaca ccaagcctcc tttcttccac 1680
   tggcaaggtg aggggcttgg tatagtttgg ggagatcccc tgactattca gtaggagaag 1740
   aaatcaaaaa tccattcttt tctccttctc tccctccaac agtggccagg ggaaggggaa 1800
   gtgagggcag gggcaaaaag atttgggaat ttttatttat ttatttattg tgacttttca 1860
   tttttttggt atttggcttt actggaatag gagggcccct gcccactgtg ccccgtttat 1920
   cccttattcc ccaaaccctg ctctccccaa cacctactca cttaagcact tgtataaagc 1980
   ctccagggtt gggaatggga gtaaagggca agagggcgga cacatgaagt ttagtttcta 2040
   acccatcatc accctaactc aaccttttct gagccaaatg gcttgaattg aagccagttg 2100
   tcatggaaat agtaagaggt tagggtttaa gagctgggga tgcgggggtg ggagagagaa 2160
   ccctcaacat ccaggatcta tataatgaga gctactttaa accctcaggt ccaccctcat 2220
   gatgctgagt tatttagcca gagggtgcag cctgcttatg cccaaattcc ctcagccaag 2280
   agagagacca aagagcctct ggaatggccc tgctcccagc ctctatcttc aggtcaatta 2340
   gagagagtat agagacccca gagtcccctg ggtctggaaa gcgttaggag aggtcaagaa 2400
   aggagcagta aggaggctga aggttacagg gcatttgaat ccaaatcact gctctgggct 2460
   agggaataga gccagcagac caaggtggga aggattctgg aagggggaca ttttagtctc 2520
   ctaaccccaa agctcagggt ggaagagggg agaacaagga agcagagtgt ataattattt 2580
   tttcctttta tttttggaat ctaacagtac ctggcagcag ggaggggaaa gtacagtggg 2640
   gaaaagcatc tgacaaggcc agttagaaca gaggatggga aggatggaga ctcccgggct     2700
   tggaaggcta ggaagcaggc agagactggt tgccatttca agtcactagc taggcccatt     2760
   cattcctccc acaaccctga cccattctcc tctggactca ctgtgcctca gtttcttccc     2820
   ctcaatggaa tgagaaatga cagcacccgc cacagccaag agatgaattc tgagcactta     2880
   ccacgggcac tttatggaca taaaatacct ctcgctgtgg gacagataac cagggcacca     2940
   gagtagtggt gaagagatgt gaggcttaag aggagtcaca ggcttcagag tacaagttcc     3000
   cctctgcctc ccagctggac agtgcctaga agccaaggag ttgagaatct cctgatccac     3060
   accctatcct tacttcacca ccaggcctct tggctccagg caagagctta gaggatgtca     3120
   ggagaggtgg gggtaagaat cttcagcaaa actgtcactc taagtagagc cagcagttac     3180
   gggtctgata aaaacagtac tgaactaaag taaagcccaa gctggtgagc aaaactggat     3240
   ggctcattct tcccaagagc atgactctcc cccttggcca gttggtggaa ggggcaaagg     3300
   tatgtgacca cccttgagaa ggtgatgttg gtgagcttta acatcttatt cctattctta     3360
   tagtgagaaa gtgaaacaag atctttcagt agaggaatgg gcagggctgt taggctcttc     3420
   agcttgcctt cacccatata gcagctatgc taaccccaag cctctctggc cctgttcttc     3480
   atccttcctt ctgccccaat cctgaaggac aagacacacc cggccatcaa caccactcac     3540
   atttccttgg tggaaggaaa ggaacagaga agtgaagaac agatacctcc ctccaaggtc     3600
   aaatgcctcg tgatcttggc agagtaggga ttgggcaata agcatcaggt atcttccctc     3660
   tacagattct agagagctgg ggcattaaat atgggggaca cttagaatac agctccttaa     3720
   ataccaccaa ataaagacct ttgtgtgtgt gtggtgggtg gggggggggc aggggtcttt     3780
   ctcttatgaa cataaatctg tgagctgaag tctcattccc ctgttcctcc ctacccccaa     3840
   agaggcacag agtgaaggga cttggggggc acagctcagc aacccagtgg gagttagcac      3900
   cccctcccac cttatgatgt gtgtggacct ggccagtgcc cctctgaaca tatcattatt     3960
   agtgtaatta tcatttattt tgtgtatttg tcacattgtg tgcatgacag cctttgttaa     4020
   gggtgtctga ggagtatgga gctgacaggg gcattggaat gccaggaaag aacttcttca     4080
   actgagatca aggcttcctg gagggaacca ctgcaaaaag gccatcaggc agttttcaag     4140
   ttatgtgaca gagggcaaag acggccatag ggtgctctga gttttgggat ggtcacatga     4200
   cacaatccag cacttgaacc tgaaaaaaaa aataaaagcg gtcaaagagt ttagaattca     4260

## Description

### BACKGROUND OF THE INVENTION

Age-dependent dysfunction in adult stem cells is attributable to both cell-intrinsic and -extrinsic inputs. Critical mechanisms underlying the functional decline of aged stem cells remain elusive. Accordingly, there exists a need to identify factors that are able to promote or reverse age-associated changes in tissues as diverse as the skeletal muscle, liver and CNS (Wagers and Conboy, Cell 122: 659 (2005); Ruckh et al., Cell Stem Cell 10:96 (2005).

### SUMMARY OF THE INVENTION

According to the present invention there is provided an agent or composition which increases the level of GDF11 polypeptide in a subject, thereby increasing neurogenesis in the subject, for use in treating or preventing a neurodegenerative disorder in the subject, wherein the agent or composition comprises a Growth and Differentiation Factor 11 (GDF11) polypeptide of SEQ ID NO: 3.

In some embodiments of the agent or composition for use, increasing neurogenesis is associated with increased neural cell proliferation, increased neural cell differentiation, increased number or frequency of neural stem cells, increased number of neural progenitor cells, increased number of neural precursor cells, and increased expression of at least one synaptic plasticity gene, at least one neuroprotective gene, or at least one neuronal specification gene, or increased number of new neurons; optionally wherein the at least one synaptic plasticity gene, at least one neuroprotective gene, and/or at least one neuronal specification gene is selected from the group consisting of neuronal PAS domain protein 4 (Npas4), dual specificity phosphatase 1 (Dusp1), heat shock 70kDa protein 8 (Hspa8), BTG family, member 2 (Btg2), activity-regulated cytoskeleton-associated protein (Arc), nuclear receptor subfamily 4, group A, member 1 (Nr4al), cysteine-rich, angiogenic inducer, 61 (Cyr61), FBJ murine osteosarcoma viral oncogene homolog (Fos), jun B proto-oncogene (Junb), Kruppel-like factor 10 (K1f10), nuclear receptor subfamily 4, group A, member 2 (Nr4a2), claudin 1 (Cldn1), early growth response 2 (Egr2), crystalline, mu (Crym), early growth response 1 (Egrl), and polio-like kinase 2 (Plk2).

In some embodiments of the agent or composition for use, neurogenesis is increased in the subventricular zone of the lateral ventricles.

In some embodiments of the agent or composition for use, neurogenesis is increased in the olfactory bulb; optionally wherein increased neurogenesis results in improved olfactory behavior.

In some embodiments of the agent or composition for use, the subject has been diagnosed with a neurodegenerative disorder due to aging.

In some embodiments of the agent or composition for use, the level of GDF11 polypeptide is increased in at least one of the subject's systemic circulation, cerebral vasculature, cerebral tissue, cerebrospinal fluid, lateral ventricles, neurovasculature, and subventricular zone neurovascular niche.

In some embodiments of the agent or composition for use, the agent or composition comprises GDF11 polypeptide homodimers comprising polypeptides of the amino acid sequence of SEQ ID NO: 3.

In one aspect, the present invention provides a pharmaceutical composition comprising a GDF11 polypeptide of SEQ ID NO: 3, which increases the level of GDF11 polypeptide in a subject, thereby increasing neurogenesis in the subject, and a pharmaceutically acceptable carrier, for use in treating or preventing a neurodegenerative disorder in the subject.

The practice of the present invention will typically employ, unless otherwise indicated, conventional techniques of cell biology, cell culture, molecular biology, transgenic biology, microbiology, recombinant nucleic acid (e.g., DNA) technology, immunology, and RNA interference (RNAi) which are within the skill of the art. Non-limiting descriptions of certain of these techniques are found in the following publications: Ausubel, F., et al., (eds.), Current Protocols in Molecular Biology, Current Protocols in Immunology, Current Protocols in Protein Science, and Current Protocols in Cell Biology, all John Wiley & Sons, N.Y., edition as of December 2008; Sambrook, Russell, and Sambrook, Molecular Cloning: A Laboratory Manual, 3rd ed., Cold Spring Harbor Laboratory Press, Cold Spring Harbor, 2001; Harlow, E. and Lane, D., Antibodies - A Laboratory Manual, Cold Spring Harbor Laboratory Press, Cold Spring Harbor, 1988; Freshney, R.I., "Culture of Animal Cells, A Manual of Basic Technique", 5th ed., John Wiley & Sons, Hoboken, NJ, 2005. Non-limiting information regarding therapeutic agents and human diseases is found in Goodman and Gilman's The Pharmacological Basis of Therapeutics, 11th Ed., McGraw Hill, 2005, Katzung, B. (ed.) Basic and Clinical Pharmacology, McGraw-Hill/Appleton & Lange; 10th ed. (2006) or 11th edition (July 2009). Non-limiting information regarding genes and genetic disorders is found in McKusick, V.A.: Mendelian Inheritance in Man. A Catalog of Human Genes and Genetic Disorders. Baltimore: Johns Hopkins University Press, 1998 (12th edition) or the more recent online database: Online Mendelian Inheritance in Man, OMIM™. McKusick-Nathans Institute of Genetic Medicine, Johns Hopkins University (Baltimore, MD) and National Center for Biotechnology Information, National Library of Medicine (Bethesda, MD), as of May 1, 2010, World Wide Web URL: http://www.ncbi.nlm.nih.gov/omim/ and in Online Mendelian Inheritance in Animals (OMIA), a database of genes, inherited disorders and traits in animal species (other than human and mouse), at http://omia.angis.org.au/contact.shtml.

Standard art-accepted meanings of terms are used herein unless indicated otherwise. Standard abbreviations for various terms are used herein.

### BRIEF DESCRIPTION OF THE DRAWINGS

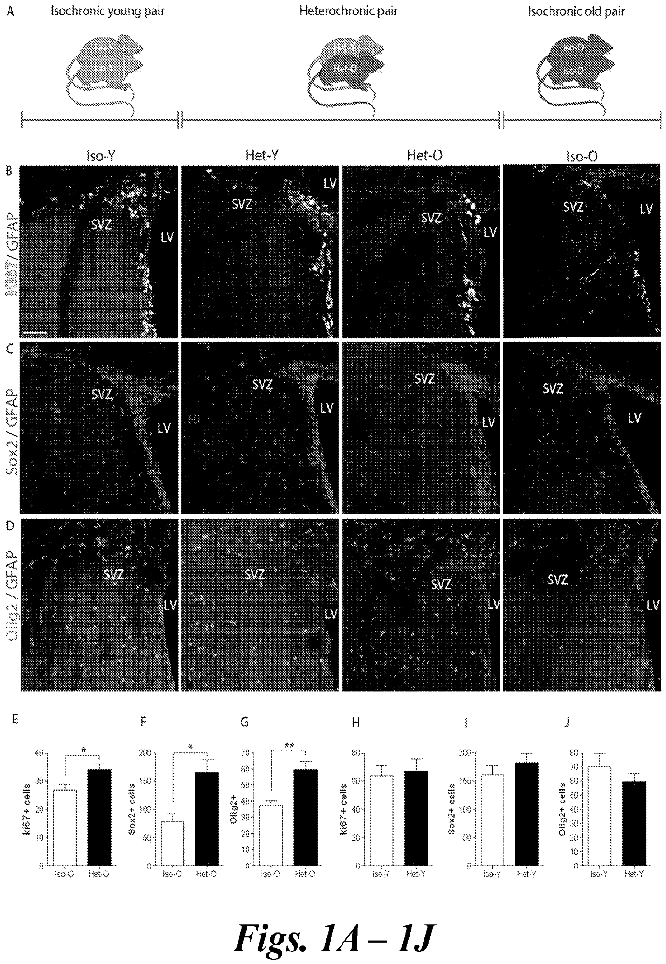

**Figs. 1A-1J** demonstrate that young blood stimulates neural stem and progenitor cell proliferation and expansion in the subventricular zone (SVZ). **Fig. 1A** shows a graphical scheme representing parabiotic pairs of young (2 months, CD45.1) and old (15-18 months, CD45.2) mice that were joined for 5 weeks. **Figs. 1B, 1C****,** and **ID** show confocal images of coronal sections of the SVZ, double-labeled for Ki67 and GFAP **(Fig. IB),** Sox2 and GFAP **(****Fig. 1C****)** and Olig2 and GFAP **(****Fig. 1D****).** Scale bar: 50µm. **Figs. IE, 1F, 1G, 1H, 1I,** and **1J** show quantification of proliferative, neural stem and progenitor cell populations in the SVZ area, depicting the effect of parabiosis between isochronic and heterochronic parabionts. Quantification of Ki67⁺ **(Fig. IE),** Sox2⁺ **(****Fig. 1F****)** and Olig2⁺ cells **(****Fig. 1G****)** in old parabionts. Quantification of Ki67⁺ **(****Fig. 1H****),** Sox2⁺ **(****Fig. 1I****)** and Olig2⁺ cells **(****Fig. 1J****)** in young parabionts (n Iso-O = 6-8, n Iso-Y = 6, n Het-O = 7-8, n Het-Y = 6, *p≤0.05, **p≤0.01). Data shown as mean ± S.E.M, statistical analysis by t-test.

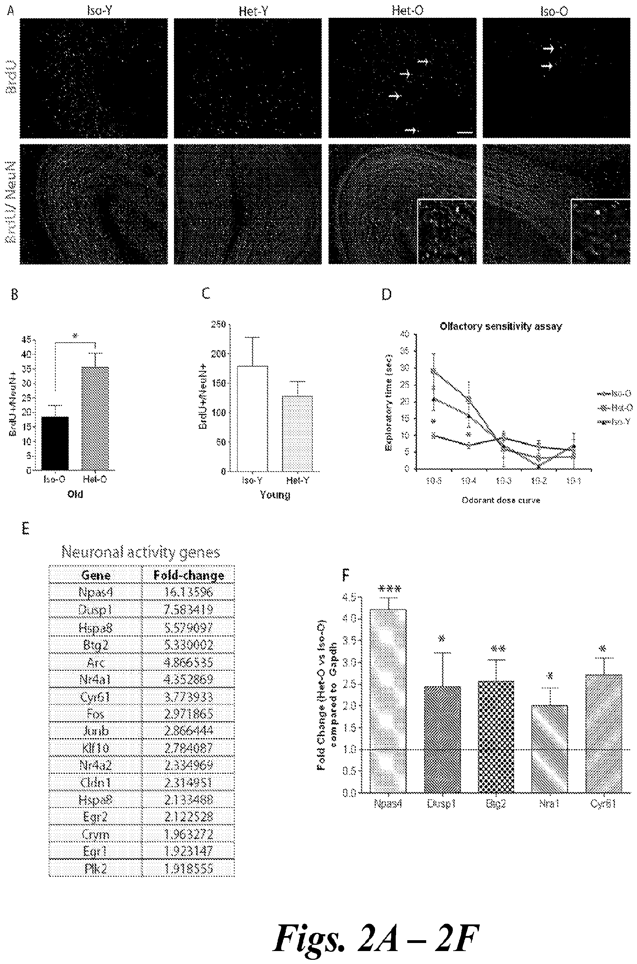

**Figs. 2A-2F** demonstrate that heterochronic parabiosis enhances neurogenesis and cognitive functions in the old mouse. **Fig. 2A** shows the maximum projection of Z-stack images of olfactory bub (OB) sections immunostained for BrdU and NeuN showing the increase in newborn neurons after heterochronic parabiosis in old mice whereas young mice are not affected. Scale bar: 100µm. **Figs. 2B** and **2C** show quantification of newborn neurons in the OB of old **(****Fig. 2B****)** and young **(****Fig. 2C****)** parabionts (n=4, *p≤0.05). Numbers are shown per field. **Fig. 2D** shows measurement of the exploratory time during the olfaction assay (n=3, **p≤0.01). Data shown as mean ± S.E.M, statistical analysis by t-test. **Fig. 2E** shows the results of microarray analysis in tabular format showing the fold-increase up-regulation of genes in Het-O compared to Iso-O that were also up-regulated in the Iso-Y versus Iso-O comparison. **Fig. 2F** shows histograms showing the fold increase in gene expression after real-time RT-PCR.

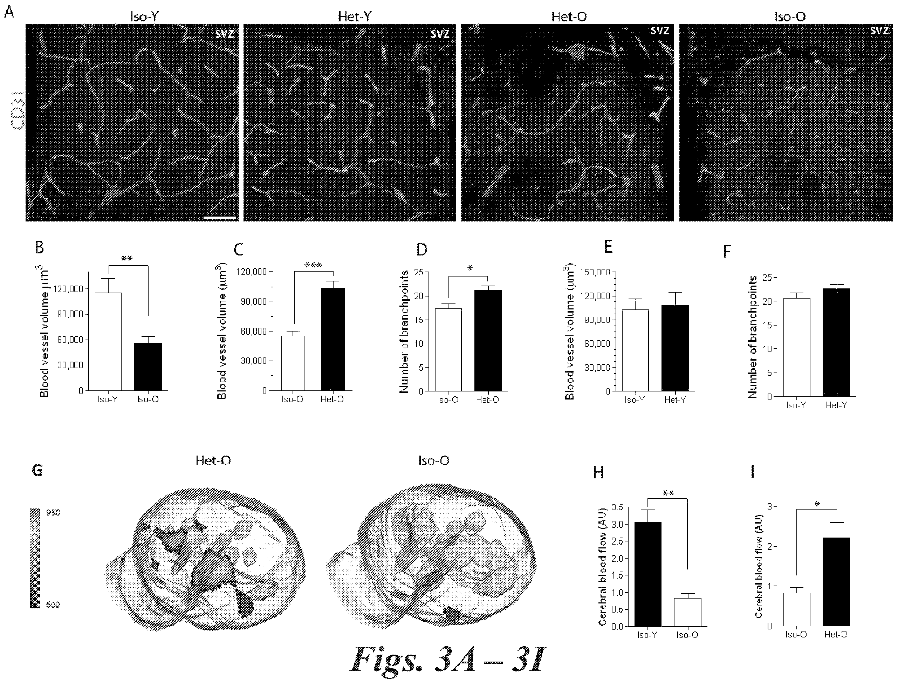

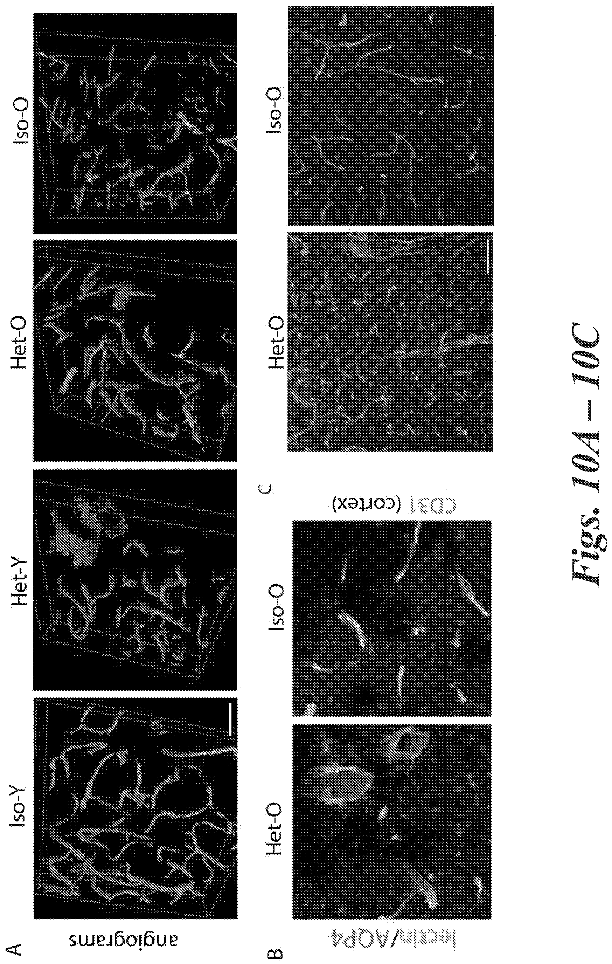

**Figs. 3A-3I** demonstrate that young blood enhances vascularization and increases blood flow in the old mouse. **Fig. 3A** shows confocal images of coronal subventricular zone (SVZ) sections stained with CD31. Scale bar: 50µm. **Figs. 3B, 3C,** and **3E** are bar graphs showing the results of volumetric analyses of 3D angiograms generated from z-stacks of 100µm thick sections (also see **Fig. 10****)** of old-young **(****Fig. 3B****),** old **(****Fig. 3C****)** and young **(****Fig. 3E****)** parabionts (n old = 9, n young = 6, ***p≤0.001). **Figs. 3D** and **3F** are bar graphs showing the quantification of blood vessel branchpoints per field in old **(****Fig. 3D****)** and young **(****Fig. 3F****)** parabionts (n old = 8, n young = 5, *p≤0.05). **Fig. 3G** shows perfusion MRI images of the SVZ. **Figs. 3H** and **3I** are bar graphs showing measurements of cerebral blood flow in Iso-O and Iso-Y **(****Fig. 3G****)** or Het-O **(****Fig. 3I****)** mice from MRI perfusion experiments (n=4, *p≤0.05, **p≤0.01). Data shown as mean ± S.E.M, statistical analysis by t-test.

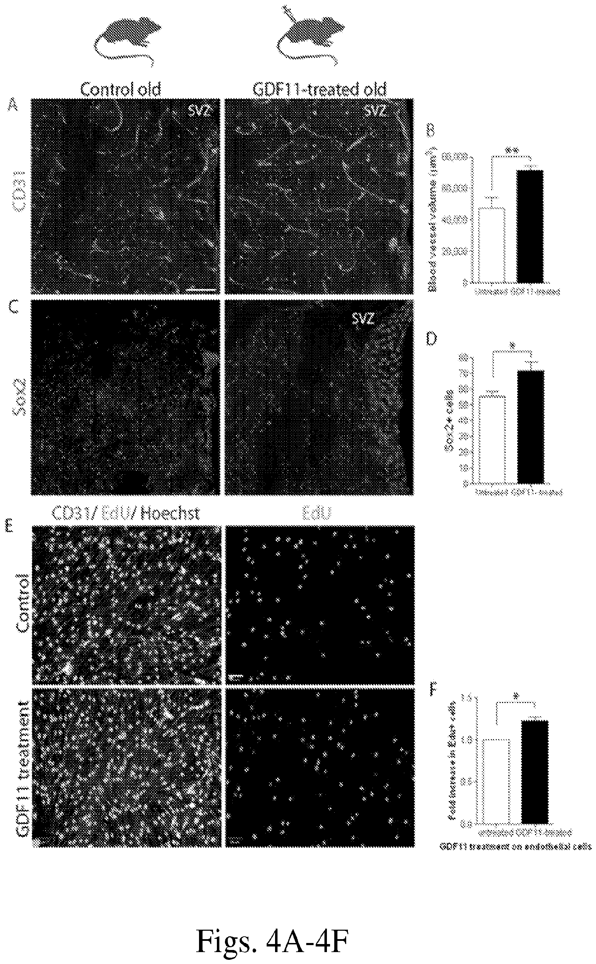

**Figs. 4A-4F** demonstrate that young circulating factor GDF11 recapitulates the effect of heterochronic parabiosis by enhancing angiogenesis and neurogenesis. **Figs. 4A** and **4C** show confocal images of coronal subventricular zone (SVZ) sections showing that old mice (22 months) injected with GDF11 for 4 weeks have enhanced vascularization marked by CD31 **(****Fig. 4A****)** as well as increased Sox2⁺ neural stem cell populations **(****Fig. 4C****)** compared to control. **Fig. 4B** is a bar graph showing the results of volumetric analyses of 3D angiograms generated from z-stacks of 100um thick sections showing that GDF11 treatment enhances blood vessel volume (n=9, **p≤0.01). **Fig. 4D** shows the quantification of Sox2⁺ cells in the SVZ area (n=6, *p≤0.05). **Fig. 4E** shows images of primary brain endothelial cell culture after treatment with or without GDF11 for 6 days and EdU for the last 4 hours, labeled with CD31, EdU and Hoechst. **Fig. 4F** is a bar graph showing quantification of the images depicted in **Fig. 4E** (n=3, *p≤0.05). Data shown as mean ± S.E.M, statistical analysis by pair-wise t-test. Scale bars: 50µm.

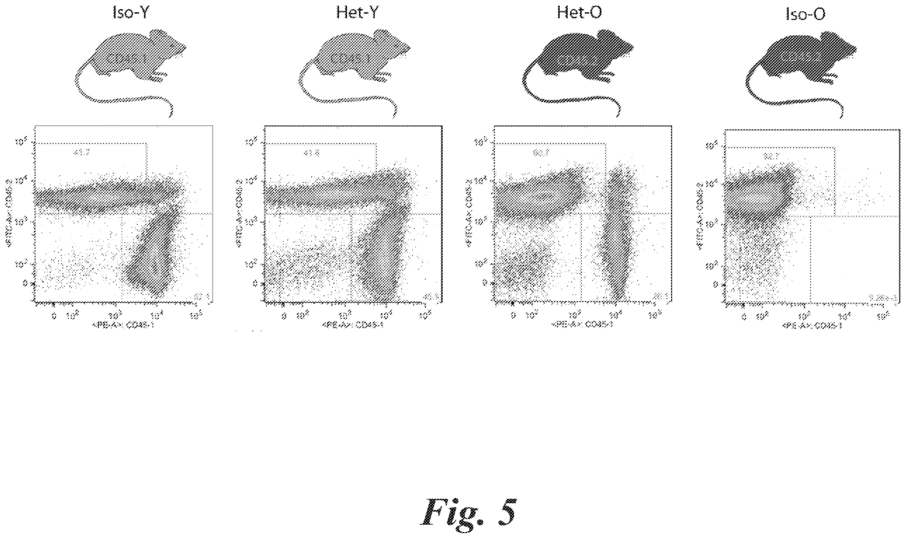

**Fig. 5** illustrates the confirmation of cross-circulation by blood chimerism by flow-cytometry analysis of the chimeric blood from Iso-Y, Het-Y, Het-O and Iso-O mice.

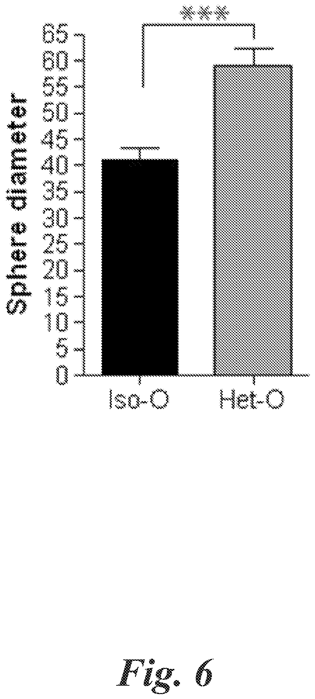

**Fig. 6** is a histogram showing the difference in neurosphere size of neurospheres derived by either Iso-O or Het-O mice and passaged at least once (n=15, **p≤0.01). Data shown as mean ± S.E.M, statistical analysis by t-test.

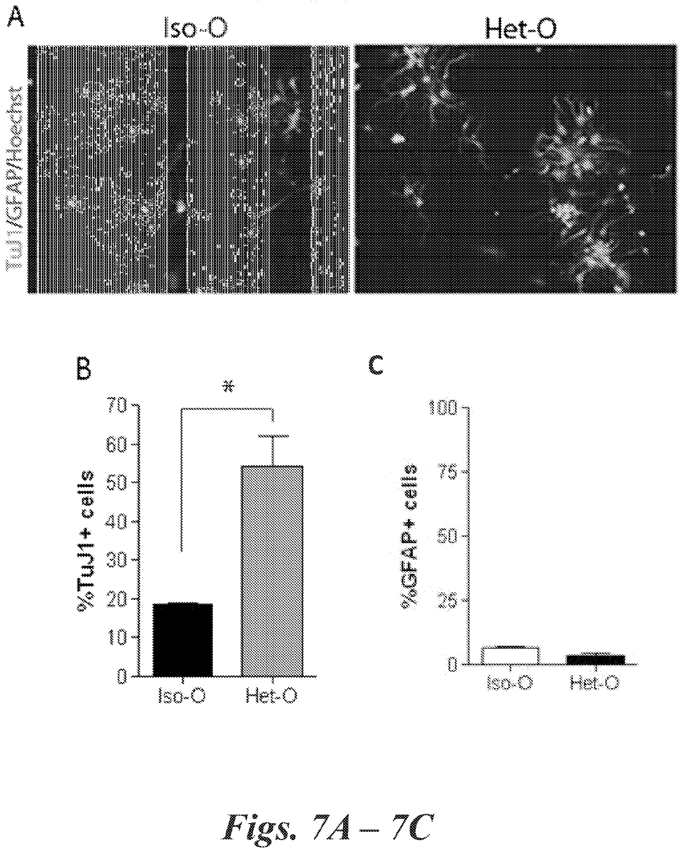

**Figs. 7A-7C** demonstrate that heterochronic parabiosis induces neuronal differentiation *in vitro.* **Fig. 7A** is a representative image showing the results of a neurosphere differentiation assay of Iso-O and Het-O derived neurospheres. **Fig. 7B** is a histogram showing the difference in TuJ1⁺ neurons. **Fig. 7C** is a histogram showing that no difference was observed in GFAP⁺ cell populations. (n=12, *p≤0.05). Data shown as mean ± S.E.M, statistical analysis by t-test.

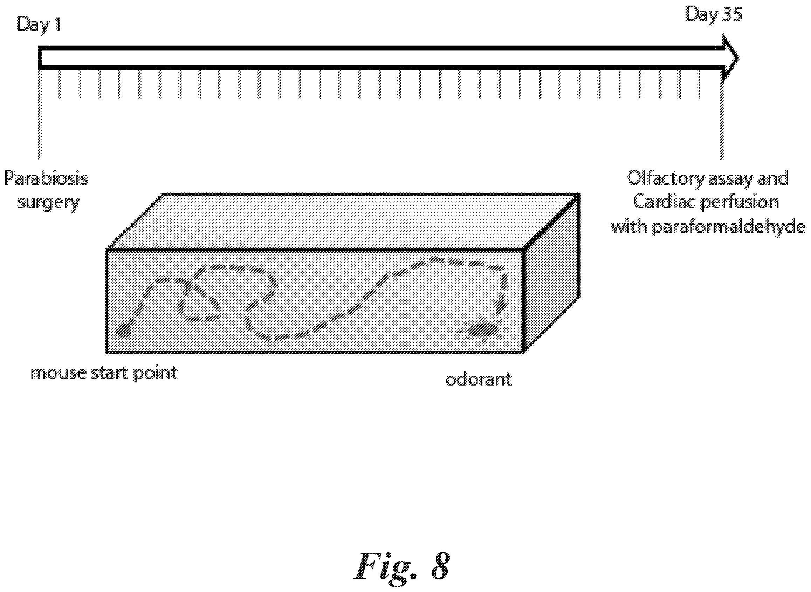

**Fig. 8** shows an experimental setup for the olfaction assay. At the end of the 5-week parabiosis, mice are placed in a clean cage and exposed to the odorant.

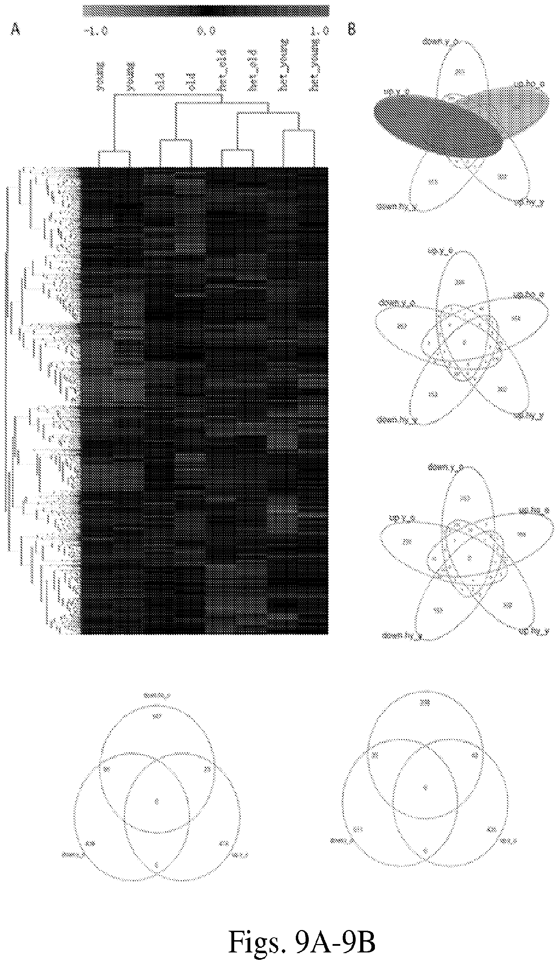

**Figs. 9A** and **9B** illustrate the results of microarray analyses on the forebrain of isochronic and heterochronic mice. **Fig. 9A** illustrates a microarray heat map and **Fig. 9B** illustrates Venn diagrams showing the numbers of genes identified in clusters that were either up-regulated or down-regulated. Note that the colored sections correspond to the list of genes on identified in **Fig. 2E****.**
**Figs. 10A-10C** depict comparisons of the vasculature of Het-O and Iso-O paired mice. **Fig. 10A** depicts 3D reconstructions of the subventricular zone (SVZ) vasculature generated by confocal imaging of 100µm thick sections and processed with VOLOCITY software. **Fig. 10B** shows confocal images of coronal SVZ sections showing double-labeling with lectin and aquaporin-4 (AQP4). **Fig. 10C** shows confocal images of coronal cortical sections labeled with CD31.

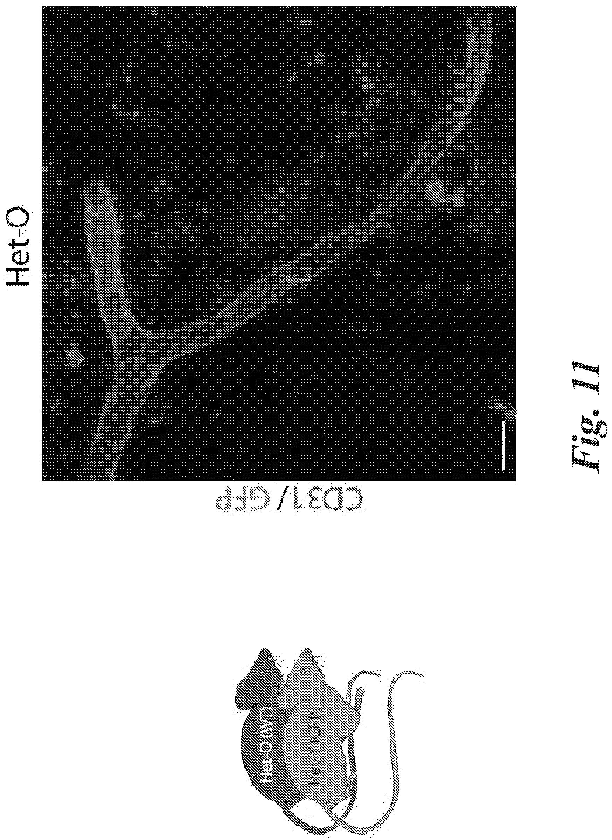

**Fig. 11** shows a comparison of heterochronic parabiosis between young- GFP mice and old non-GFP mice. High magnification confocal image of the Het-O showing that there is no co-localization of circulating GFP progenitors with CD31⁺ (red) blood vessels.

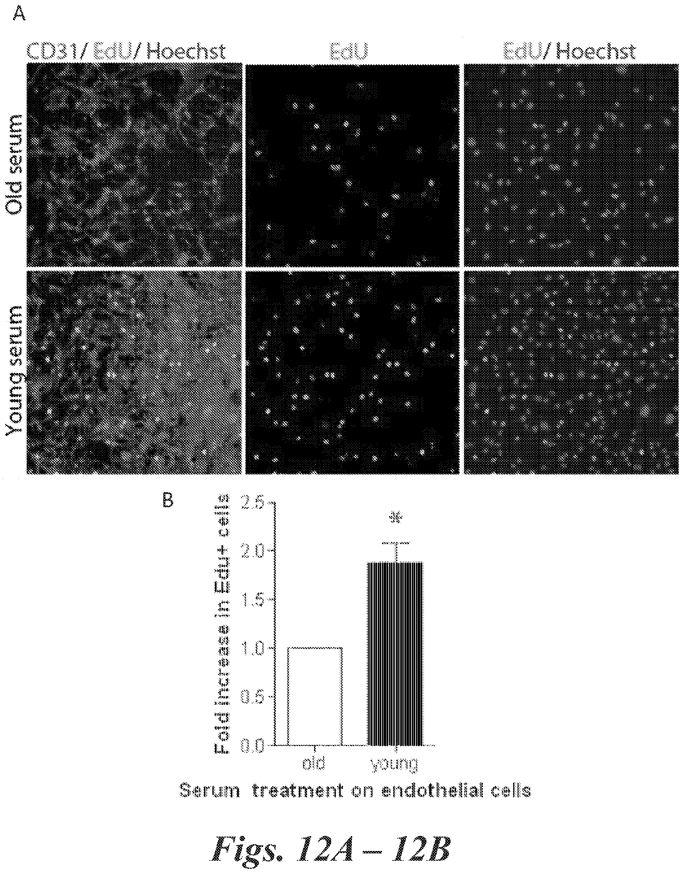

**Figs. 12A** and **12B** demonstrate the effects of either young or old mouse serum on primary brain capillary endothelial cells. **Fig. 12A** shows representative images of primary brain capillary endothelial cell cultures treated with serum-free media supplemented with either young or old mouse serum for 2 days and then pulsed for 4 hours with EdU. Scale bar: 50 µm. **Fig. 12B** is a bar graph showing the quantification of **Fig. 12A****.** (n=3, *p≤0.05). Data shown as mean ± S.E.M, statistical analysis by pair-wise t-test.

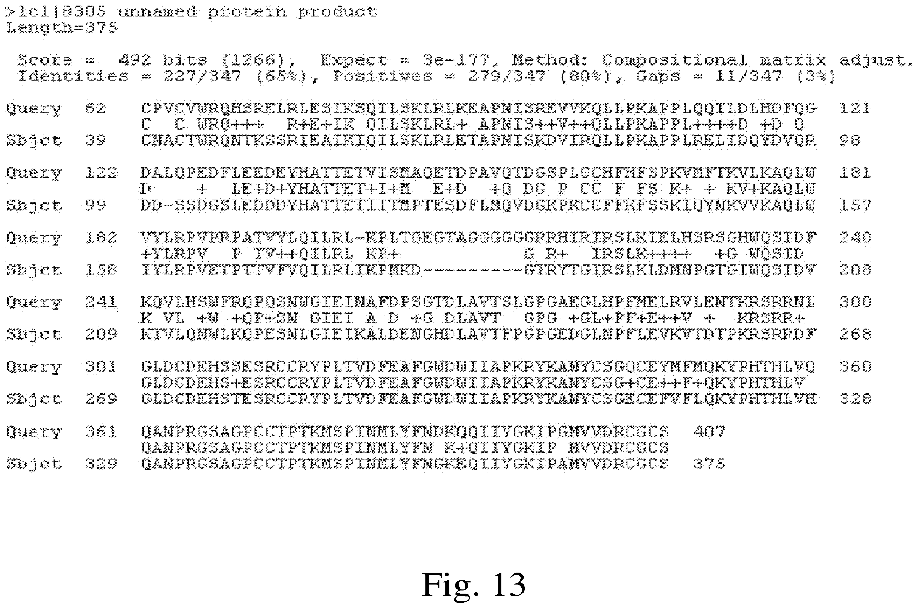

**Fig. 13** depicts an example of an alignment between human GDF11 precursor peptide (query sequence; residues 62-407 of SEQ ID NO: 1) and human GDF8 precursor peptide created using the default settings of the alignment tool of the BLASTP program.

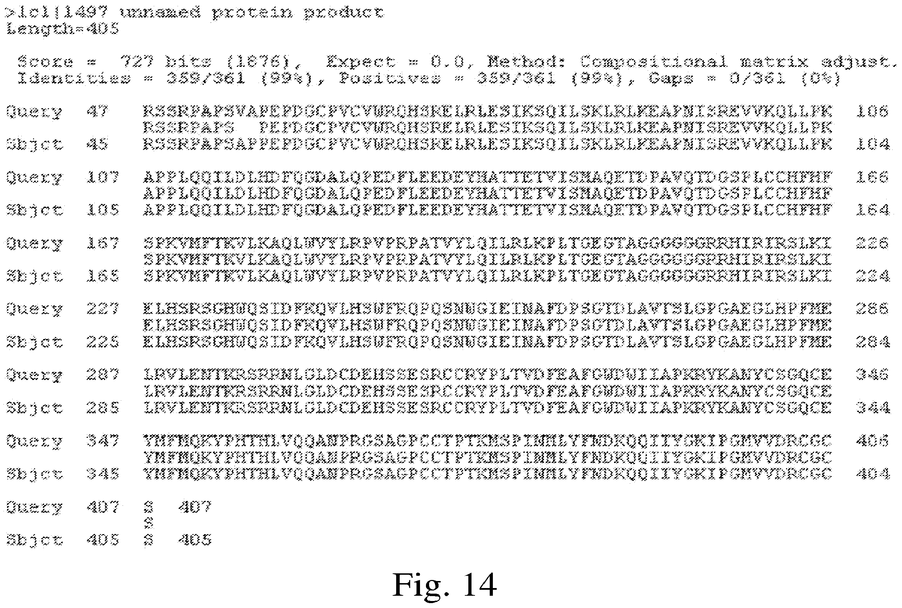

**Fig. 14** depicts an example of an alignment between human GDF11 precursor peptide (query sequence; residues 47-407 of SEQ ID NO: 1) and murine GDF11 precursor peptide created using the default settings of the alignment tool of the BLASTP program.

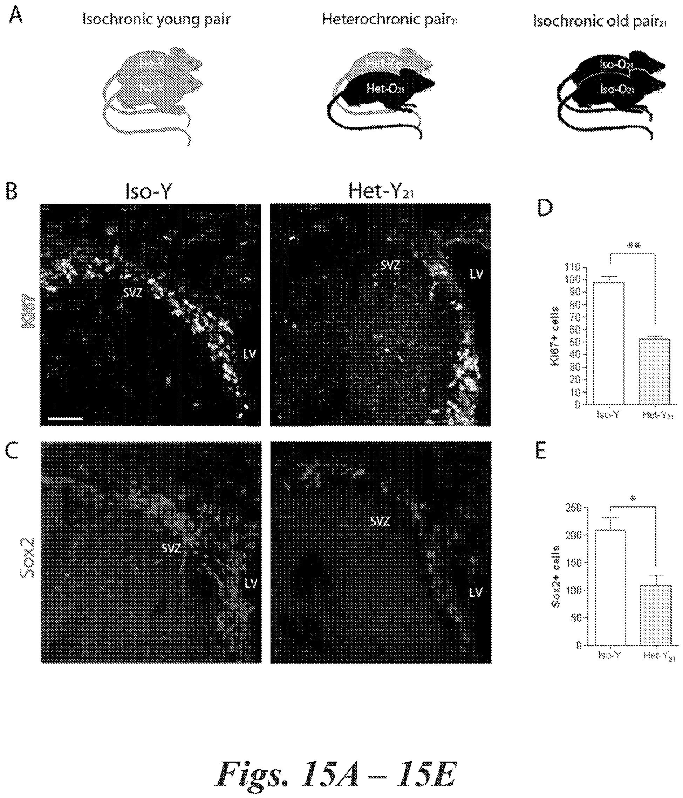

**Figs. 15A-15E** illustrate an age-dependent accumulation of factors in the blood of older mice that affect neurogenic zones in both the hippocampus and subventricular zone (SVZ) of the young mice. **Fig. 15A** graphically depicts parabiotic pairs of young (2 months, CD45.1) and old (21 months, CD45.2) mice that were joined for 5 weeks. **Figs. 15B** and **C** show confocal images of coronal sections of the SVZ, labeled for Ki67 **(****Fig. 15B****)** and Sox2 **(****Fig. 15C****).** Scale bar: 50µm. **Figs. 15D** and **E** are bar graphs respectively showing the quantification of Ki67⁺ cells and Sox2⁺ cells in the SVZ area of young parabionts (n = 4 animals per condition , *p≤0.05, **p≤0.01). Data shown as mean ± S.E.M; statistical analysis by Student's t-test.

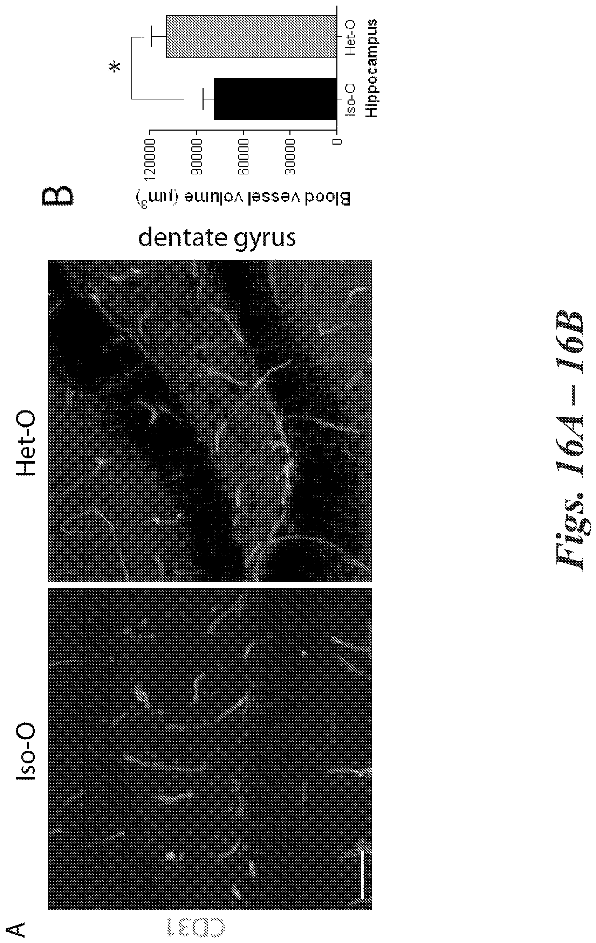

**Figs. 16A** and **16B** illustrate that increased vascularization in the Het-O mice extended to other neurogenic areas like the hippocampus. **Fig. 16A** shows confocal images of coronal sections of hippocampus dentate gyrus labeled with CD31. Scale bar: 50µm. **Fig. 16B** illustrates the results of a volumetric analysis of 3D angiograms generated from z-stacks of 100µm thick sections of the dentate gyrus (n = 7 animals per condition, *p≤0.05). Data shown as mean ± S.E.M; statistical analysis by Student's t-test.

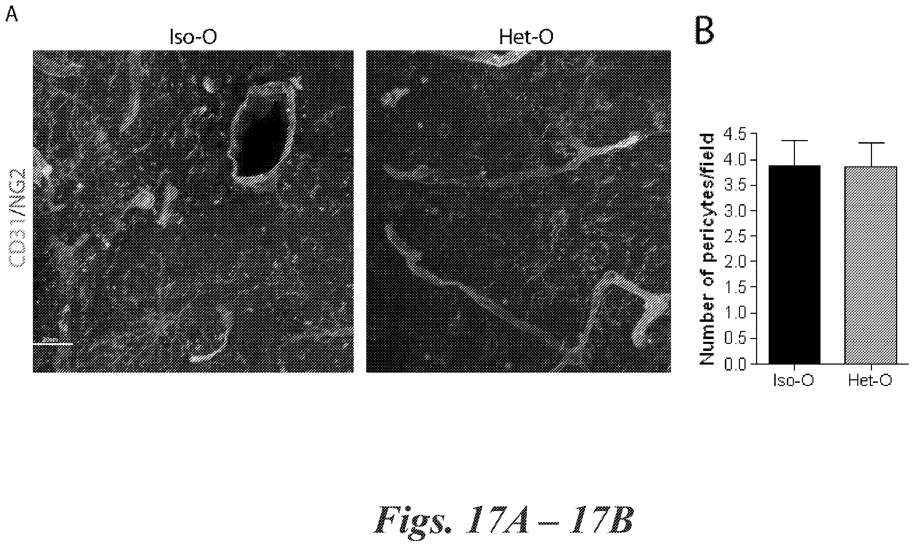

**Figs. 17A** and **17B** show that the number of pericytes, which play a role in capillary vasoconstriction, were unaltered by heterochronic parabiosis. **Fig. 17A** illustrates confocal images of the subventricular zone (SVZ) coronal sections double-labeled with NG2 and CD31 to mark pericytes. Scale bar: 20µm. **Fig. 17B** shows the quantification of NG2⁺ /CD31⁺ cells in the SVZ area of old parabionts (n = 4 animals per condition). Data shown as mean ± S.E.M; statistical analysis by Student's t-test.

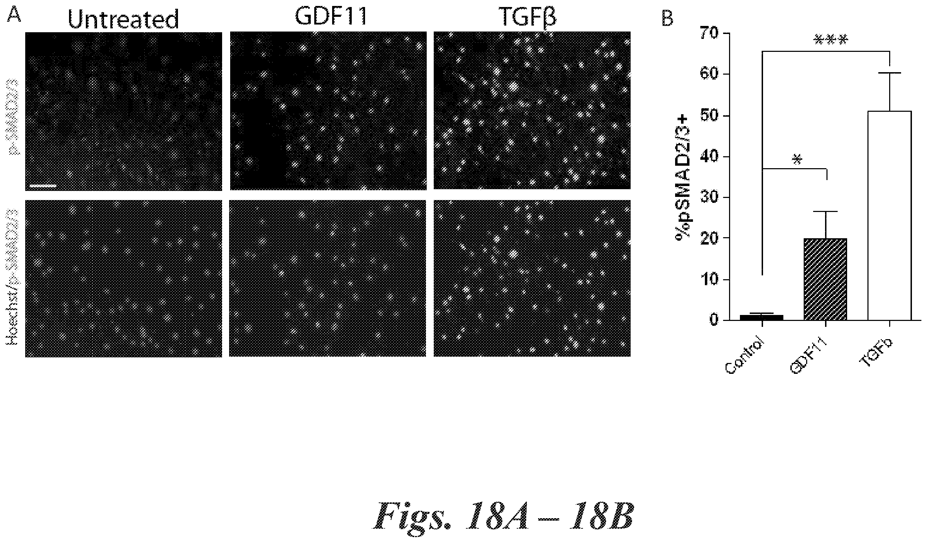

**Figs. 18A** and **18B** illustrate that GDF11 recapitulates the effect of heterochronic parabiosis by enhancing vascular remodeling and neurogenesis. **Fig. 18A** shows representative images of primary brain capillary endothelial cell cultures treated with either GDF11 (40ng/ml) or TFGβ (10ng/ml) in the presence of the control sodium orthovanadate to preserve phosphorylation for 30 minutes. Scale bar: 100 µm. **Fig. 18B** shows the quantification of the images presented in **Fig. 18A** (n=7, *p≤0.05). Data shown as mean ± S.E.M; statistical analysis by t-test, compared to the control.

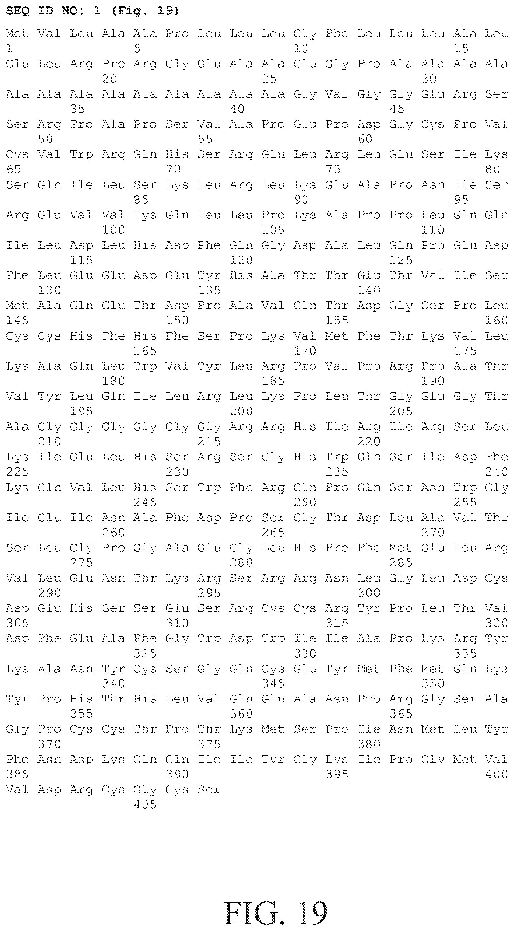

**Fig. 19** shows the amino acid sequence encoding a human GDF11 precursor polypeptide (SEQ ID NO: 1).

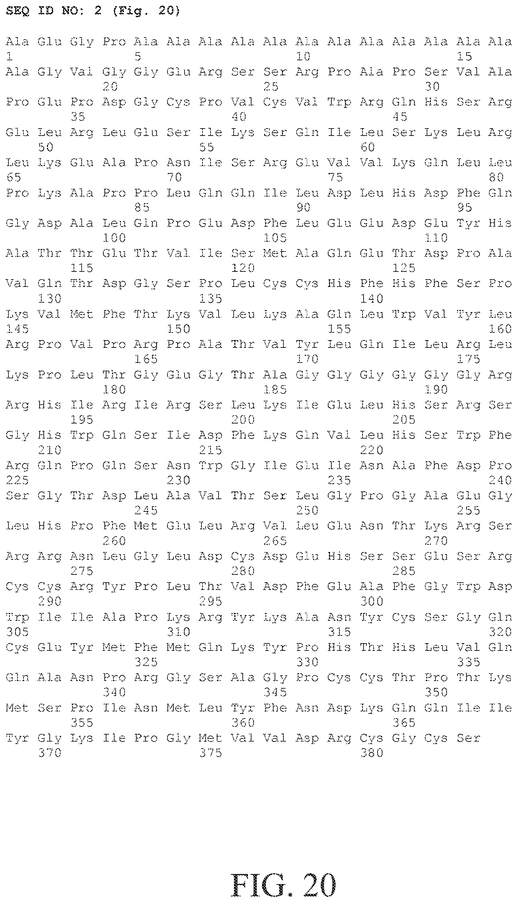

**Fig. 20** shows the amino acid sequence encoding a human GDF11 pro-peptide (SEQ ID NO: 2).

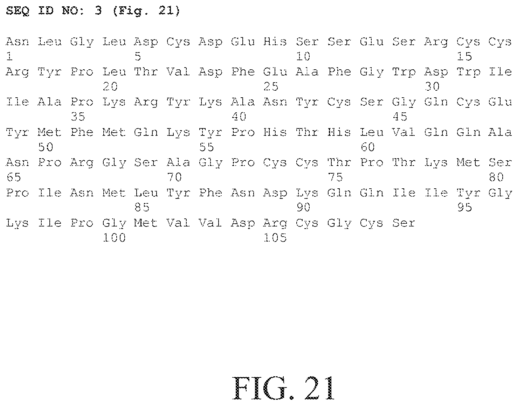

**Fig. 21** shows the amino acid sequence encoding a human mature GDF11 polypeptide (SEQ ID NO: 3).

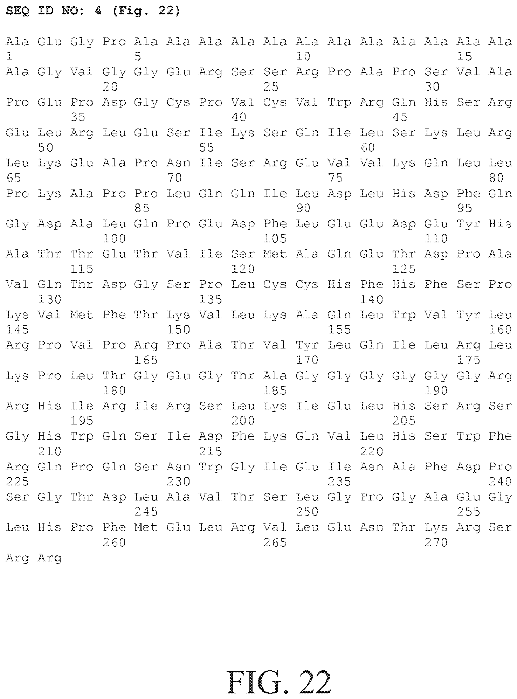

**Fig. 22** shows the amino acid sequence encoding a human GDF11 N-terminal polypeptide (SEQ ID NO: 4),

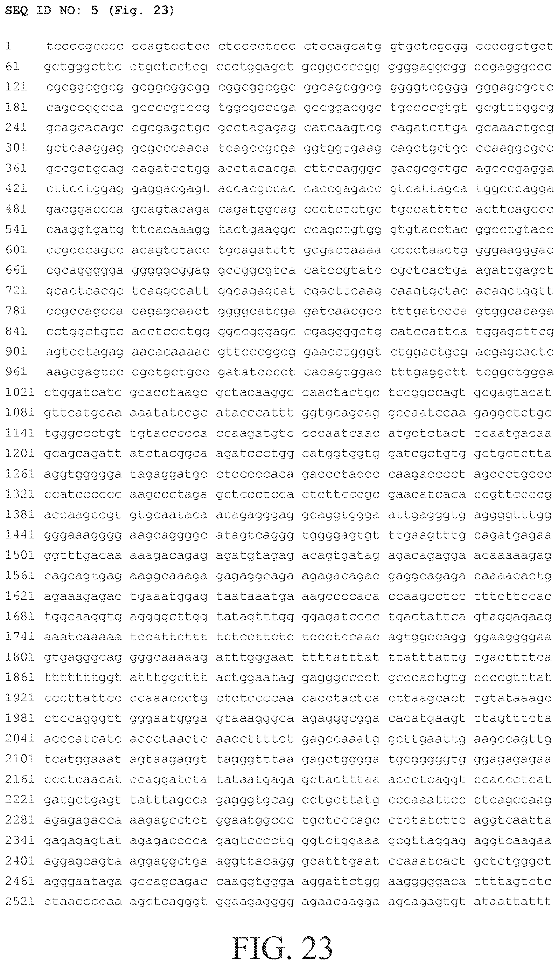

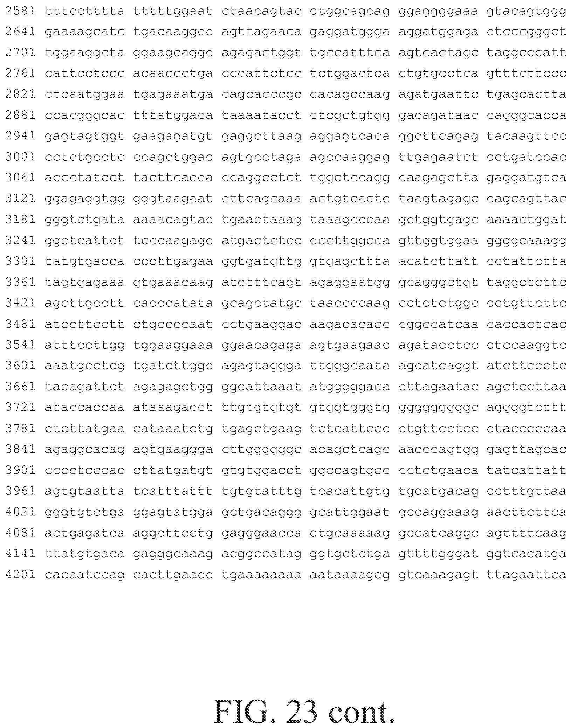

**Fig. 23** shows the nucleic acid sequence encoding human GDF11 mRNA (SEQ ID NO: 5).

### DETAILED DESCRIPTION OF THE INVENTION

Described herein are methods and compositions based on the discovery that as animals age, the level of GDF11 polypeptide in their blood decreases and deterioration of neural stem cells and/or progenitor cells increases, accompanied by a diminished capacity for neurogenesis and angiogenesis. The methods and compositions described herein are useful for rejuvenating the neurovascular niche (e.g., neural stem cells and/or progenitor cells), reversing decline (e.g., age-related decline) in neurogenesis and/or angiogenesis, increasing neurogenesis, increasing angiogenesis, treating or preventing neurodegenerative disorders, treating or preventing neurovascular disorders, for example, neurodegenerative disorders or neurovascular disorders associated with decreased neurogenesis and angiogenesis, respectively, due to aging. The methods and compositions described herein are also useful for increasing neuronal activity, inducing vascular remodeling, increasing neuroplasticity, increasing cognitive functioning, increasing olfactory sensitivity/behavior, or increasing neuronal stem cell and/or stem cell progenitor numbers and/or proliferation rate. The methods and compositions described herein generally relate to increasing the level of GDF11 (e.g., GDF11 polypeptide) in a subject to treat, prevent, or reverse the neurodegenerative and neurovascular disorders, conditions, and symptoms described herein. In particular embodiments the compositions described herein have effect in the subventricular zone of the brain, while as described herein they may have effect in one or more non-neurogenic regions of the brain.

For convenience, certain terms employed herein are collected here. Unless stated otherwise, or implicit from context, the following terms and phrases include the meanings provided below. Unless explicitly stated otherwise, or apparent from context, the terms and phrases below do not exclude the meaning that the term or phrase has acquired in the art to which it pertains. The definitions are provided to aid in describing particular embodiments, and are not intended to limit the claimed invention. Unless otherwise defined, all technical and scientific terms used herein have the meaning commonly understood by one of skill in the art.

As used herein, "neurodegenerative disorder" refers to a disease condition involving neuronal loss mediated or characterized at least partially by at least one of deterioration of neural stem cells and/or progenitor cells, a decreased capacity for neurogenesis, or a reduction in circulating GDF11 polypeptide in a subject. Non-limiting examples of neurodegenerative disorders include polyglutamine expansion disorders (e.g., HD, dentatorubropallidoluysian atrophy, Kennedy's disease (also referred to as spinobulbar muscular atrophy), and spinocerebellar ataxia (e.g., type 1, type 2, type 3 (also referred to as Machado-Joseph disease), type 6, type 7, and type 17)), other trinucleotide repeat expansion disorders (e.g., fragile X syndrome, fragile XE mental retardation, Friedreich's ataxia, myotonic dystrophy, spinocerebellar ataxia type 8, and spinocerebellar ataxia type 12), Alexander disease, Alper's disease, Alzheimer disease, amyotrophic lateral sclerosis (ALS), ataxia telangiectasia, Batten disease (also referred to as Spielmeyer-Vogt-Sjogren-Batten disease), Canavan disease, Cockayne syndrome, corticobasal degeneration, Creutzfeldt-Jakob disease, Guillain-Barre syndrome, ischemia stroke, Krabbe disease, kuru, Lewy body dementia, multiple sclerosis, multiple system atrophy, non-Huntingtonian type of Chorea, Parkinson's disease, Pelizaeus-Merzbacher disease, Pick's disease, primary lateral sclerosis, progressive supranuclear palsy, Refsum's disease, Sandhoff disease, Schilder's disease, spinal cord injury, spinal muscular atrophy (SMA), SteeleRichardson-Olszewski disease, and Tabes dorsalis.

In certain contexts, neurodegenerative disorders encompass neurological injuries or damages to the CNS or the PNS associated with physical injury (e.g., head trauma, mild to severe traumatic brain injury (TBI), spinal cord injury, diffuse axonal injury, craniocerebral trauma, cranial nerve injuries, cerebral contusion, intracerebral haemorrhage and acute brain swelling), ischemia (e.g., resulting from spinal cord infarction or ischemia, ischemic infarction, stroke, cardiac insufficiency or arrest, atherosclerotic thrombosis, ruptured aneurysm, embolism or haemorrhage), certain medical procedures or exposure to biological or chemic toxins or poisons (e.g., surgery, coronary artery bypass graft (CABG), electroconvulsive therapy, radiation therapy, chemotherapy, anti-neoplastic drugs, immunosuppressive agents, psychoactive, sedative or hypnotic drugs, alcohol, bacterial or industrial toxins, plant poisons, and venomous bites and stings), tumors (e.g., CNS metastasis, intraaxial tumors, primary CNS lymphomas, germ cell tumors, infiltrating and localized gliomas, fibrillary astrocytomas, oligodendrogliomas, ependymomas, pleomorphic xanthoastrocytomas, pilocytic astrocytomas, extraaxial brain tumors, meningiomas, schwannomas, neurofibromas, pituitary tumors, and mesenchymal tumors of the skull, spine and dura matter), infections (e.g., bacterial, viral, fungal, parasitic or other origin is selected from the group consisting of pyrogenic infections, meningitis, tuberculosis, syphilis, encephalomyelitis and leptomeningitis), metabolic or nutritional disorders (e.g., glycogen storage diseases, acid lipase diseases, Wemicke's or Marchiafava-Bignami's disease, Lesch-Nyhan syndrome, Farber's disease, gangliosidoses, vitamin B12 and folic acid deficiency), cognition or mood disorders (e.g., learning or memory disorder, bipolar disorders and depression), and various medical conditions associated with neural damage or destruction (e.g., asphyxia, prematurity in infants, perinatal distress, gaseous intoxication for instance from carbon monoxide or ammonia, coma, hypoglycaemia, dementia, epilepsy and hypertensive crises).

As used herein, "neurovascular disorder" refers to any disease or condition that results in cerebrospinal ischema, infarction, and hemorrhage mediated or characterized at least partially by a decreased capacity for angiogenesis or a reduction in circulating GDF11 polypeptide in a subject. Neurovascular disorders encompass any abnormality of the blood vessels within or supplying the brain and spine. This includes narrowing of arteries, which reduces blood flow to the brain and increases the risk of stroke (particularly "ischemic" stroke), and weakening of arteries, which may create brain aneurysms and increases the risk of intracranial bleeding (or "hemorrhagic" stroke.) Non-limiting examples of neurovascular disorders include brain atherothrombosis, brain aneurysms, brain arteriovenous malformations, brain embolism, brain ischemia, for example caused by atherothrombosis, embolism, or hemodynamic abnormalities, cardiac arrest, carotid stenosis, cerebrovascular spasm, headache, intracranial hemorrhage, ischemic stroke, seizure, spinal vascular malformations, reflex neurovascular dystrophy (RND), neurovascular compression disorders such as hemifacial spasms, tinnitus, trigeminal neuralgia, glossopharyngeal neuralgia, stroke, transient ischemic attacks, and vasculitis.

The terms "decrease," "reduce," "reduced," "reduction," "decrease," and "'inhibit" are all used herein generally to mean a decrease by a statistically significant amount relative to a reference. However, for avoidance of doubt, "reduce," "reduction" or "decrease" or "inhibit" typically means a decrease by at least 10% as compared to a reference level and can include, for example, a decrease by at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 98%, at least about 99% , up to and including, for example, the complete absence of the given entity or parameter as compared to the reference level, or any decrease between 10-99% as compared to the absence of a given treatment.

The terms "increased," "increase" or "enhance" or "activate" are all used herein to generally mean an increase by a statically significant amount; for the avoidance of any doubt, the terms "increased", "increase" or "enhance" or "activate" means an increase of at least 10% as compared to a reference level, for example an increase of at least about 20%, or at least about 30%, or at least about 40%, or at least about 50%, or at least about 60%, or at least about 70%, or at least about 80%, or at least about 90%, or up to and including a 100% increase or any increase between 10-100% as compared to a reference level, or at least about a 2-fold, or at least about a 3-fold, or at least about a 4-fold, or at least about a 5-fold or at least about a 10-fold increase, or any increase between 2-fold and 10-fold or more as compared to a reference level.

The term "isolated" or "partially purified" as used herein refers, in the case of a nucleic acid or polypeptide, to a nucleic acid or polypeptide separated from at least one other component (*e.g*., nucleic acid or polypeptide) that is present with the nucleic acid or polypeptide as found in its natural source and/or that would be present with the nucleic acid or polypeptide when expressed by a cell, or secreted in the case of secreted polypeptides. A chemically synthesized nucleic acid or polypeptide or one synthesized using *in vitro* transcription/translation is considered "isolated."

The term "biological sample" as used herein denotes a sample taken or isolated from a biological organism, e.g., cerebral tissue, cerebrospinal fluid, blood sample, cell lysate, a homogenate of a tissue sample from a subject, or a fluid sample from a subject. Exemplary biological samples include, but are not limited to, brain biopsies, cerebrospinal fluid, or blood and/or serum samples. The sample may be from a resection, biopsy, or core needle biopsy. In addition, fine needle aspirate samples can be used. Samples can include paraffin-embedded and frozen tissue. The term "biological sample" also includes untreated or pretreated (or pre-processed) biological samples. The biological sample may be an untreated biological sample. The sample can be obtained by removing a sample of cells from a subject, but can also be accomplished by using previously isolated cells (e.g. isolated at a prior time point and isolated by the same or another person).

As used herein, a "subject" means a human or animal. Usually the animal is a vertebrate such as a primate, rodent, domestic animal or game animal. Primates include chimpanzees, cynomologous monkeys, spider monkeys, and macaques, e.g., Rhesus. Rodents include mice, rats, woodchucks, ferrets, rabbits and hamsters. Domestic and game animals include cows, horses, pigs, deer, bison, buffalo, feline species, e.g., domestic cat, canine species, e.g., dog, fox, wolf, avian species, e.g., chicken, emu, ostrich, and fish, e.g., trout, catfish and salmon. Patient or subject includes any subset of the foregoing, e.g., all of the above, but excluding one or more groups or species such as humans, primates or rodents. The subject may be a mammal, e.g., a primate, e.g., a human. The terms, "patient", "individual" and "subject" are used interchangeably herein. Preferably, the subject is a mammal. The mammal can be a human, non-human primate, mouse, rat, dog, cat, horse, or cow, but are not limited to these examples. Mammals other than humans can be advantageously used, for example, as subjects that represent animal models of, for example, neurodegenerative or neurovascular disorders (e.g., stroke). In addition, the methods described herein can be used to treat domesticated animals and/or pets. A subject can be male or female. A subject can be one who has been previously diagnosed with or identified as suffering from or having a condition in need of treatment (e.g., a neurodegenerative or neurovascular disorder, e.g., due to decreased capacity for neurogenesis or angiogenesis) or one or more complications related to such a condition, and optionally, but need not have already undergone treatment for a condition or the one or more complications related to the condition. Alternatively, a subject can also be one who has not been previously diagnosed as having a condition in need of treatment or one or more complications related to such a condition. Rather, a subject can include one who exhibits one or more risk factors for a condition or one or more complications related to a condition. A "subject in need" of treatment for a particular condition can be a subject having that condition, diagnosed as having that condition, or at increased risk of developing that condition relative to a given reference population.

As used herein the term "comprising" or "comprises" is used in reference to compositions, methods, and respective component(s) thereof, that are essential to the method or composition, yet open to the inclusion of unspecified elements, whether essential or not.

The term "consisting of' refers to compositions, methods, and respective components thereof as described herein, which are exclusive of any element not recited in that description of the embodiment.

As used herein the term "consisting essentially of' refers to those elements required for a given embodiment. The term permits the presence of elements that do not materially affect the basic and novel or functional characteristic(s) of that embodiment.

The term "statistically significant" or "significantly" refers to statistical significance and generally means a "p" value greater than 0.05 (calculated by the relevant statistical test). Those skilled in the art will readily appreciate that the relevant statistical test for any particular experiment depends on the type of data being analyzed. Additional definitions are provided in the text of individual sections below.

Definitions of common terms in cell biology and molecular biology can be found in "The Merck Manual of Diagnosis and Therapy", 19th Edition, published by Merck Research Laboratories, 2006 (ISBN 0-911910-19-0); RobertS. Porter et al. (eds.), The Encyclopedia of Molecular Biology, published by Blackwell Science Ltd., 1994 (ISBN 0-632-02182-9); The ELISA guidebook (Methods in molecular biology 149) by Crowther J. R. (2000); Immunology by Werner Luttmann, published by Elsevier, 2006. Definitions of common terms in molecular biology can also be found in Benjamin Lewin, Genes X, published by Jones & Bartlett Publishing, 2009 (ISBN-10: 0763766321); Kendrew et al. (eds.), Molecular Biology and Biotechnology: a Comprehensive Desk Reference, published by VCH Publishers, Inc., 1995 (ISBN 1-56081-569-8) and Cun-ent Protocols in Protein Sciences 2009, Wiley Intersciences, Coligan et al., eds.

Unless otherwise stated, the present invention was performed using standard procedures, as described, for example in Sambrook et al., Molecular Cloning: A Laboratory Manual (3 ed.), Cold Spring Harbor Laboratory Press, Cold Spring Harbor, N.Y., USA (2001) and Davis et al., Basic Methods in Molecular Biology, Elsevier Science Publishing, Inc., New York, USA (1995).

Described herein are methods comprising administering to a subject an agent or composition which increases the level of GDF11 polypeptide in the subject.

The subject may be one who is in need of rejuvenated neural stem cells and/or progenitor cells. As used herein "rejuvenated neural stem cells and/or progenitor cells" and "rejuvenating neural stem and/or progenitor cells" refer to a restoration of the neural stem cells and/or progenitor cells either partially or wholly to the quality and/or quantity of neural stem cells and/or progenitor cells typically found within a youthful healthy subject of the same species. The number of neural stem cells and/or progenitor cells may be restored completely to the number neural stem cells and/or progenitor cells of a youthful healthy subject of the same species. The number of neural stem cells and/or progenitor cells in the subject may be restored to within 95%, to within 90%, to within 85%, to within 80%, to within 75%, to within 70%, to within 65%, to within 60%, to within 55%, or to within 50% of the number of neural stem cells and/or progenitor cells of a youthful subject. In some contexts, "rejuvenated neural stem cells and/or progenitor cells" and "rejuvenating neural stem cells and/or progenitor cells" refers to an increase in the number or frequency of the neural stem cells and/or progenitor cells" in the subject compared to a number or frequency of the neural stem and/or progenitor cells before administration of an agent or composition which increased the level of GDF11 polypeptide in the subject. The number or frequency of neural stem cells and/or progenitor cells may be increased by at least 10% in the subject. The number or frequency of neural stem cells and/or progenitor cells may be increased by at least 15% in the subject. The number or frequency of neural stem cells and/or progenitor cells may be increased by at least 20% in the subject. The number or frequency of neural stem cells and/or progenitor cells may be increased by at least 25% in the subject. The number or frequency of neural stem cells and/or progenitor cells may be increased by at least 30% in the subject. The number or frequency of neural stem cells and/or progenitor cells may be increased by at least 35% in the subject. The number or frequency of neural stem cells and/or progenitor cells may be increased by at least 40% in the subject. The number or frequency of neural stem cells and/or progenitor cells may be increased by at least 45% in the subject. The number or frequency of neural stem cells and/or progenitor cells may be increased by at least 50% in the subject. The number or frequency of neural stem cells and/or progenitor cells may be increased by at least 60%, at least 65%, at least 70%, at least 75%, at least 80%, or at least 90% in the subject. The number or frequency of neural stem cells and/or progenitor cells may be increased by at least 100% in the subject. The number or frequency of neural stem cells and/or progenitor cells may be increased by at least 1.1 fold, at least 1.2 fold, at least 1.3 fold, at least 1.4 fold, at least 1.5 fold, at least 1.6 fold, at least 1.7 fold, at least 1.8 fold, at least 1.9 fold, at least 2 fold, at least 3 fold, at least 4 fold, at least 5 fold, at least 10 fold or more in the subject.

The subject may be one who is in need of increased neurogenesis. The subject may be one who has, or has been diagnosed as having a neurodegenerative disorder. The subject may be one who has, or has been diagnosed as having a neurodegenerative disorder characterized by decreased neurogenesis. The subject may be one who has, or has been diagnosed as having a neurodegenerative disorder characterized at least partially by a decreased capacity for neurogenesis due to aging. The subject is one who is at risk of developing a neurodegenerative disorder due to aging. The subject may be one who has, or has been diagnosed as having, or is at risk of developing, dementia. The subject may be one who has, or has been diagnosed as having, or is at risk of developing, Alzheimer's disease. The subject may be one who has, or has been diagnosed as having, or is at risk of developing, mild cognitive impairment. The subject may be one who has, or has been diagnosed as having, or is at risk of developing, depression. The subject may be one who has, or has been diagnosed as having, or is at risk of developing, a learning disorder. The subject may be one who has, or has been diagnosed as having, or is at risk of developing, a memory disorder.

The subject may be one who is in need of increased angiogenesis. The subject may be one who has, or has been diagnosed as having a neurovascular disorder. The subject may be one who has, or has been diagnosed as having a neurovascular disorder characterized by decreased angiogenesis. The subject may be one who has, or has been diagnosed as having a neurovascular disorder characterized at least partially by a decreased capacity for angiogenesis due to aging. The subject may be one who is at risk of developing a neurovascular disorder due to aging. The subject may be one who has, or has been diagnosed as having, or is at risk of developing, stroke. The subject may be one who has, or has been diagnosed as having, or is at risk of developing, ischemia.

The subject may be an elderly subject. An elderly subject may be one that is over the age of 50, 55, 60, 65, 70, 75, 80, 85, 90, or 100 years.

The agent or composition which increases the level of GDF11 polypeptide may be administered to a subject who has or has been diagnosed with a neurodegenerative disorder described herein. A health care professional may diagnose a subject as having a neurodegenerative disorder by the assessment of one or more symptoms of a neurodegenerative disorder in the subject. Non-limiting symptoms of a neurodegenerative disorder in a subject include difficulty lifting the front part of the foot and toes; weakness in arms, legs, feet, or ankles; hand weakness or clumsiness; slurring of speech; difficulty swallowing; muscle cramps; twitching in arms, shoulders, and tongue; difficulty chewing; difficulty breathing; muscle paralysis; partial or complete loss of vision; double vision; tingling or pain in parts of body; electric shock sensations that occur with head movements; tremor; unsteady gait; fatigue; dizziness; loss of memory; disorientation; misinterpretation of spatial relationships; difficulty reading or writing; difficulty concentrating and thinking; difficulty making judgments and decisions; difficulty planning and performing familiar tasks; depression; anxiety; social withdrawal; mood swings; irritability; aggressiveness; changes in sleeping habits; wandering; dementia; loss of automatic movements; impaired posture and balance; rigid muscles; bradykinesia; slow or abnormal eye movements; involuntary jerking or writhing movements (chorea); involuntary, sustained contracture of muscles (dystonia); lack of flexibility; lack of impulse control; and changes in appetite. A health care professional may also base a diagnosis, in part, on the subject's family history of a neurodegenerative disorder. A health care professional may diagnose a subject as having a neurodegenerative disorder upon presentation of a subject to a health care facility (e.g., a clinic or a hospital). In some instances, a health care professional may diagnose a subject as having a neurodegenerative disorder while the subject is admitted in an assisted care facility. Typically, a physician diagnoses a neurodegenerative disorder in a subject after the presentation of one or more symptoms.

The agent or composition which increases the level of GDF11 polypeptide may be administered to a subject who has or has been diagnosed with a neurological injury or damage to the CNS or the PNS associated with physical injury, ischemia, certain medical procedures or exposure to biological or chemic toxins or poisons, tumors, infections, metabolic or nutritional disorders, cognition or mood disorders, and various medical conditions associated with neural damage or destruction described herein.

The agent or composition which increases the level of GDF11 polypeptide may be administered to a subject who has or has been diagnosed with a neurovascular disorder described herein. A health care professional may diagnose a subject as having a neurovascular disorder by the assessment of one or more symptoms of a neurovascular disorder in the subject. Non-limiting symptoms of a neurovascular disorder in a subject include hypertension, platelet aggregation, altered cerebrovascular architecture (e.g., narrowing, stiffness, deformation), sudden blood pressure shifts, facial weakness, visual impairment, loss of coordination, or balance, a sudden headache, and mental confusion with unintelligible speech.

The agent or composition which increases the level of GDF11 polypeptide may be administered to a subject who is at risk of developing a neurovascular disorder described herein. A subject who is at risk of developing a neurovascular disorder includes individuals who are elderly, have a history of diabetes, smoking, or ischemic heart disease, for example.

The level of GDF11 polypeptide may be the level of GDF11 in (a) the systemic circulation of a subject; (b) the cerebral vasculature of a subject; (c) the neurovasculature of a subject; (d) the cerebral tissue of a subject; (e) the cerebrospinal fluid of a subject; (f) the lateral ventricles of a subject; and/or (g) the subventricular zone neurovascular niche of a subject.

The level of GDF11 in a subject can be determined by obtaining a biological sample from the subject and determining the level of GDF11 in the biological sample. Methods for determining the level of a polypeptide in a subject or a sample obtained from a subject are well known in the art and include, but are not limited to, ELISA, radioimmunoassay, immunohistochemistry, methods involving a labeled antibody specific for GDF11, dot blot analysis, functional bioassays, Northern blot, in-situ hybridization, and RT-PCR, aptamer-based proteomic technology (e.g., SOMAscan™ commercially available from SomaLogic, Inc.) among others. Antibodies specific for GDF11 are commercially available, e.g. Cat. No. ab71347 from Abeam: Cambridge, MA. The antibodies may be antibodies which do not cross-react with GDF8. The antibodies may be selective GDF11 monoclonal antibodies. The level of GDF11 polypeptide may be determined by measuring the level of an mRNA encoding a GDF11 polypeptide. The level of GDF11 can be measured as described in Souza et al., Molecular Endocrinology 2008 22:2689-2702.

As animals age, neural stem cell and/or progenitor cells deteriorate (e.g., decrease in number or function) and animals exhibit diminished capacity for neurogenesis and decreased neuroplasticity, which is often accompanied with cognitive decline associated with one or more neurodegenerative disorders (e.g., dementia). Without wishing to be bound by theory, it is believed that deterioration of neural stem and/or progenitor cells and characteristic attendant reduced neurogenesis results in part from decreased levels of circulating GDF11 polypeptide. Surprisingly, the work described herein demonstrates that aged mice treated *in vivo* with daily IP injection of recombinant GDF11 (rGDF11) caused the subject's neural stem cells and/or progenitor cells to proliferate, which was accompanied by increased neurogenesis and angiogenesis.

In particular, the rGDF11 treatment increased the number of Sox2⁺ cells by 22.5% indicating increased neurogenesis, and increased cerebral blood flow by as much as 33.5%, as measured by volumetric assays of blood vessels of mice treated with GDF11 compared to PBS control.

Described herein is a method of rejuvenating neural stem cells and/or progenitor cells in a subject in need thereof, comprising administering to the subject an agent or composition which increases the level of GDF11 polypeptide in the subject. The agent or composition may cause the subject's neural stem cells and/or progenitor cells to undergo at least one of an increase in frequency or number, proliferation, differentiation, and an increase in neurogenesis.

According to the present invention, increasing neurogenesis may be associated with increased neural cell proliferation, increased neural cell differentiation, increased number or frequency of neural stem cells, increased number of neural progenitor cells, increased number of neural precursor cells, and increased expression of at least one synaptic plasticity gene, at least one neuroprotective gene, or at least one neuronal specification gene selected from the group consisting of neuronal PAS domain protein 4 (Npas4) (NCBI Gene ID: 266743), dual specificity phosphatase 1 (Dusp1) (NCBI Gene ID: 1843), heat shock 70kDa protein 8 (Hspa8) (NCBI Gene ID: 3312), BTG family, member 2 (Btg2) (NCBI Gene ID: 7832), activity-regulated cytoskeleton-associated protein (Arc) (NCBI Gene ID: 23237), nuclear receptor subfamily 4, group A, member 1 (Nr4a1) (NCBI Gene ID: 3164), cysteine-rich, angiogenic inducer, 61 (Cyr61) (NCBI Gene ID: 3491), FBJ murine osteosarcoma viral oncogene homolog (Fos) (NCBI Gene ID: 2353), jun B proto-oncogene (Junb) (NCBI Gene ID: 3726), Kruppel-like factor 10 (Klf10) (NCBI Gene ID: 7071), nuclear receptor subfamily 4, group A, member 2 (Nr4a2) (NCBI Gene ID: 4929), claudin 1 (Cldn1) (NCBI Gene ID: 9076), early growth response 2 (Egr2) (NCBI Gene ID: 1959), crystalline, mu (Crym) (NCBI Gene ID: 1428), early growth response 1 (Egr1) (NCBI Gene ID: 1958), and polio-like kinase 2 (Plk2) (NCBI Gene ID: 10769), and increasing the number of new neurons. In some embodiments, neurogenesis is increased in the subventricular zone of the lateral ventricles. In some embodiments, neurogenesis is increased in the olfactory bulb. In some embodiments, increased neurogenesis results in improved olfactory behavior.

Described herein is a method for increasing angiogenesis in a subject in need thereof, comprising administering to the subject an agent or composition which increases the level of GDF11 polypeptide in the subject, thereby increasing angiogenesis in the subject. Increasing angiogenesis may comprise increasing cerebrovascular architecture, increasing capillary density, increasing cerebral blood flow, and increasing cerebral vessel sprouting branch points.

The methods and compositions described herein relate to increasing the level of GDF11 polypeptide in a subject. As used herein, "GDF11" refers to "Growth and Differentiation Factor 11" (NCBI Gene ID No: 10220), a member of the Transforming Growth Factor-beta superfamily of growth factors. GDF11 is known to bind TGFβ3 superfamily type I receptors including ALK4, ALK5, and ALK7. For signaling in mammalian development, GDF11 predominantly uses ALK4 and ALK5. In some embodiments, GDF11 signaling can also occur via the ACVR2B receptor. GDF11 is also closely related to GDF8 (also known as myostatin). GDF11 can also be referred to as bone morphogenic protein 11, i.e. BMP11. As used herein, "GDF11" can include the human precursor polypeptide (SEQ ID NO: 1, NCBI Ref Seq: NP _005802); the human pro-peptide (SEQ ID NO: 2); the human N-terminal polypeptide (SEQ ID NO: 4), and the human mature (SEQ ID NO: 3) forms of GDF11 as well as homologs from other species, including but not limited to bovine, dog, cat, chicken, murine, rat, porcine, bovine, turkey, horse, fish, baboon and other primates. The terms also refer to fragments or variants of GDF11 that maintain at least 50% of the neurogenesis and angiogenesis increasing effect of the full length GDF11 of SEQ ID NO: 2, SEQ ID NO: 1, or SEQ ID NO: 3, e.g. as measured in an appropriate animal model (e.g., heterochronic parabiosis of aged mice).

Conservative substitution variants that maintain any of the neural stem cell and/or progenitor cell rejuvenating effect, the neurogenesis effect, or the angiogenesis effect of wild type GDF11 will include a conservative substitution as defined herein. The identification of amino acids most likely to be tolerant of conservative substitution while maintaining at least 50% of the activity of the wild type GDF11 is guided by, for example, sequence alignment with GDF11 homologs or paralogs from other species. Amino acids that are identical between GDF11 homologs are less likely to tolerate change, while those showing conservative differences are obviously much more likely to tolerate conservative change in the context of an artificial variant. Similarly, positions with non-conservative differences are less likely to be critical to function and more likely to tolerate conservative substitution in an artificial variant. Variants can be tested for activity, for example, by administering the variant to an appropriate animal model (e.g., aged mice).

For human GDF11, the pro-peptide plus signal sequence (e.g. the precursor polypeptide, e.g., SEQ ID NO: 1) is 407 amino acids long. Cleavage of the 24 amino acid signal peptide generates a pro-peptide (e.g., SEQ ID NO: 2) of 383 amino acids and cleavage of the pro-peptide results in a mature GDF11 polypeptide (e.g., SEQ ID NO: 3) of 109 amino acids that corresponds to the C-terminal 109 amino acids of the pro-peptide. The mature polypeptide forms a disulfide-linked homodimer. Cleavage of the pro-peptide also generates the N-terminal polypeptide (e.g., SEQ ID NO: 4) comprising amino acids 25-298 of SEQ ID NO: 1. The N-terminal GDF11 polypeptide can antagonize the activity of, e.g., the polypeptides of SEQ ID NOs: 2 and 3, at least *in vitro* by forming a complex with other forms of GDF11 polypeptides and can thus be used to modulate the activity of GDF11 compositions as described herein. Thus, to the extent that GDF11 polypeptides as described herein rejuvenate neural stem cells and/or progenitor cells or increase neurogenesis or angiogenesis, and to the extent the N-terminal GDF11 polypeptide of, e.g., SEQ ID NO: 4, can antagonize such effects, the polypeptide of SEQ ID NO: 4 can be excluded from the meaning of "GDF11 polypeptide" as that term is used herein.

As used herein, the terms "proteins" and "polypeptides" are used interchangeably to designate a series of amino acid residues connected to the other by peptide bonds between the alpha-amino and carboxy groups of adjacent residues. The terms "protein", and "polypeptide" refer to a polymer of protein amino acids, including modified amino acids (e.g., phosphorylated, glycated, glycosylated, etc.) and amino acid analogs, regardless of its size or function. "Protein" and "polypeptide" are often used in reference to relatively large polypeptides, whereas the term "peptide" is often used in reference to small polypeptides, but usage of these terms in the art overlaps. The terms "protein" and "polypeptide" are used interchangeably herein when refining to a gene product and fragments thereof.

Thus, exemplary polypeptides or proteins include gene products, naturally occurring proteins, homologs, orthologs, paralogs, fragments and other equivalents, variants, fragments, and analogs of the foregoing.

As used herein, "pro-peptide" used in reference to GDF11 refers to a GDF11 polypeptide in which the signal domain (e.g. amino acids 1-24 of SEQ ID NO: 1) has been cleaved off during formation of the mature and/or active forms of GDF11. As used herein, "precursor peptide" used in reference to a GDF11 polypeptide comprising the signal domain, e.g., a polypeptide comprising the amino acid sequence of SEQ ID NO: 1

The level of GDF11 in a subject may be increased by administering an agent or composition comprising a GDF11 agonist that increases the level or activity of GDF11 in the subject. Exemplary GDF11 agonists are described in U.S. Pat. No. 8,323,964.

The level of GDF11 in a subject may be increased by administering an agent or composition comprising an agonist antibody that increases expression of GDF11 in the subject. Any antibody that is capable of increasing expression of GDF11 in the subject can be used. As used herein "antibody" includes, but is not limited to, full length antibodies, antibody fragments, single chain antibodies, bispecific antibodies, minibodies, domain antibodies, synthetic antibodies (sometimes referred to herein as "antibody mimetics"), chimeric antibodies, humanized antibodies, antibody fusions (sometimes referred to as "antibody conjugates"), and fragments of each, respectively. Exemplary antibody fragments include, but are not limited to, (i) the Fab fragment consisting of VL, VH, CL and CH I domains, (ii) the Fd fragment consisting of the VH and CHI domains, (iii) the Fv fragment consisting of the VL and VH domains of a single antibody, (iv) the dAb fragment, which consists of a single variable domain, (v) isolated CDR regions, (vi) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site, (viii) bispecific single chain Fv dimers, and (ix) "diabodies" or "triabodies", multivalent or multispecific fragments constructed by gene fusion. The antibody fragments may be modified. For example, the molecules may be stabilized by the incorporation of disulfide bridges linking the VH and VL domains. Examples of antibody formats and architectures are described in Holliger & Hudson, 2006, Nature Biotechnology 23(9): 1 126-1 136, and Carter 2006, Nature Reviews Immunology 6:343-357 and references cited therein. Exemplary GDF11 agonist antibodies include the GDF11 agonist antibodies described in PCT International Application Publication WO/2002/010214.

The level of GDF11 in a subject may be increased by administering an agent or composition comprising a GDF11 polypeptide and/or a nucleic acid encoding a GDF11 polypeptide. A GDF11 polypeptide administered to a subject according to the methods described herein can comprise a GDF11 polypeptide as described herein above, e.g. a pro-peptide or mature form. The GDF11 polypeptide may comprise the amino acid sequence of SEQ ID NO: 1, SEQ ID NO: 2, or SEQ ID NO: 4. The agent for use according to the present invention is a GDF11 polypeptide comprising the amino acid sequence of SEQ ID NO: 3.

The agent or composition administered to the subject can comprise GDF11 polypeptide homodimers comprising polypeptides of the amino acid sequence of SEQ ID NO: 3, SEQ ID NO: 4, SEQ ID NO: 2, or SEQ ID NO: 1. The agent or composition administered to the subject may comprise GDF11 polypeptide heterodimers comprising polypeptides of any of the amino acid sequence of SEQ ID NO: 4, SEQ ID NO: 3, SEQ ID NO: 2, and/or SEQ ID NO: 1.

A variant or fragment of a GDF11 polypeptide can be administered to a subject. The variant of GDF11 may be a conservatively modified variant. The subject can be administered a variant or fragment (e.g. a conservatively modified variant or a functional fragment or a nucleic acid encoding such a polypeptide) of a polypeptide selected from Collectin kidney 1 (e.g. NCBI Gene ID No: 78989), Cathespin D (e.g. NCBI Gene ID No: 1509), Dickkopf-related protein 4 (e.g. NCBI Gene ID No: 27121), Erythrocyte membrane protein 4.1 (e.g. NCBI Gene ID No: 2035), esterase D (e.g. NCBI Gene ID No: 2098), hemoglobin (e.g. NCBI Gene ID No: 3043 or 3047), interleukin-1 receptor accessory protein (e.g. NCBI Gene ID No: 3556), natural killer group 2 member D (e.g. NCBI Gene ID No: 22914), Ras-related C3 botulinum toxin substrate 1 (e.g. NCBI Gene ID No: 5879), GTP-binding nuclear protein Ran (e.g. NCBI Gene ID No: 5901), tissue inhibitor of metalloproteases 3 (e.g. NCBI Gene ID No: 7078), and thymidylate synthase (e.g. NCBI Gene ID No: 7298).

The GDF11 polypeptide can be a variant of a sequence described herein, e.g. a variant of a GDF11 polypeptide comprising the amino acid sequence of SEQ ID NO: 4, SEQ ID NO: 3, SEQ ID NO: 1, or SEQ ID NO: 2. The variant may be a conservative substitution variant. Variants can be obtained by mutations of native nucleotide sequences, for example. A "variant," as referred to herein, is a polypeptide substantially homologous to a native or reference polypeptide, but which has an amino acid sequence different from that of the native or reference polypeptide because of one or a plurality of deletions, insertions or substitutions. Polypeptide-encoding DNA sequences encompass sequences that comprise one or more additions, deletions, or substitutions of nucleotides when compared to a native or reference DNA sequence, but that encodes a variant protein or fragment thereof that retains the relevant biological activity relative to the reference protein, i.e., can rejuvenate neural stem cells and/or progenitor cells at least 50% as well as wild type GDF11. As to amino acid sequences, one of skill will recognize that individual substitutions, deletions or additions to a nucleic acid, peptide, polypeptide, or protein sequence which alters a single amino acid or a small percentage, (i.e. 5% or fewer, e.g. 4% or fewer, or 3% or fewer, or 1% or fewer) of amino acids in the encoded sequence is a "conservatively modified variant" where the alteration results in the substitution of an amino acid with a chemically similar amino acid. It is contemplated that some changes can potentially improve the relevant activity, such that a variant, whether conservative or not, has more than 100% of the activity of wild type GDF11, e.g. 110%, 125%, 150%, 175%, 200%, 500%, 1000% or more.

One method of identifying amino acid residues which can be substituted is to align, for example, human GDF11 to a GDF11 homolog from one or more non-human species. Alignment can provide guidance regarding not only residues likely to be necessary for function but also, conversely, those residues likely to tolerate change. Where, for example, an alignment shows two identical or similar amino acids at corresponding positions, it is more likely that that site is important functionally. Where, conversely, alignment shows residues in corresponding positions to differ significantly in size, charge, hydrophobicity, etc., it is more likely that that site can tolerate variation in a functional polypeptide. Similarly, alignment with a related polypeptide from the same species, e.g. GDF8, which does not show the same activity, can also provide guidance with respect to regions or structures required for GDF11 activity. **Fig. 13** depicts an example of an alignment between human GDF11 precursor peptide (query sequence; residues 62-407 of SEQ ID NO: 1) and human GDF8 precursor peptide created using the default settings of the alignment tool of the BLASTP program, freely available on the world wide web at http://blast.ncbi.nlm.nih.gov/. **Fig. 14** depicts an example of an alignment between human GDF11 precursor peptide (query sequence; residues 47-407 of SEQ ID NO: 1) and murine GDF11 precursor peptide created using the default settings of the alignment tool of the BLASTP program, freely available on the world wide web at http://blast.ncbi.nlm.nih.gov/. The variant amino acid or DNA sequence can be at least 90%, at least 95%, at least 96%, at least 97%, at least 98%, at least 99%, or more, identical to a native or reference sequence, e.g. SEQ ID NO: 4, SEQ ID NO: 3, SEQ ID NO: 1, or SEQ ID NO: 2 or a nucleic acid encoding one of those amino acid sequences. The degree of homology (percent identity) between a native and a mutant sequence can be determined, for example, by comparing the two sequences using freely available computer programs commonly employed for this purpose on the World Wide Web. The variant amino acid or DNA sequence can be at least 90%, at least 91%, at least 92%, at least 93%, at least 94%, at least 95%, at least 96%, at least 97%, at least 98%, at least 99%, or more, similar to the sequence from which it is derived (referred to herein as an "original" sequence). The degree of similarity (percent similarity) between an original and a mutant sequence can be determined, for example, by using a similarity matrix. Similarity matrices are well known in the art and a number of tools for comparing two sequences using similarity matrices are freely available online, e.g. BLASTp (available on the world wide web at http://blast.ncbi.nlm.nih.gov), with default parameters set.

It is noted that the mature GDF11 polypeptide includes likely intrachain disulfide bonds between, e.g. amino acid 313 and 372; 341 and 404; and 345 and 406 (numbered relative to the full length polypeptide, including the signal sequence) and that amino acid 371 likely participates in interchain disulfide bonding.

A given amino acid can be replaced by a residue having similar physiochemical characteristics, e.g., substituting one aliphatic residue for another (such as Ile, Val, Leu, or Ala for one another), or substitution of one polar residue for another (such as between Lys and Arg; Glu and Asp; or Gln and Asn). Other such conservative substitutions, e.g., substitutions of entire regions having similar hydrophobicity characteristics, are well known. Polypeptides comprising conservative amino acid substitutions can be tested in any one of the assays described herein to confirm that a desired apoptotic activity of a native or reference polypeptide is retained. Conservative substitution tables providing functionally similar amino acids are well known in the art. Such conservatively modified variants are in addition to and do not exclude polymorphic variants, interspecies homologs, and alleles consistent with the disclosure. Typically conservative substitutions for one another include: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, e.g., Creighton, Proteins (1984)).

Any cysteine residue not involved in maintaining the proper conformation of the polypeptide also can be substituted, generally with serine, to improve the oxidative stability of the molecule and prevent aberrant crosslinking. Conversely, cysteine bond(s) can be added to the polypeptide to improve its stability or facilitate oligomerization.

The GDF11 polypeptide administered to a subject can comprise one or more amino acid substitutions or modifications. The substitutions and/or modifications can prevent or reduce proteolytic degradation and/or prolong half-life of the polypeptide in the subject. A GDF11 polypeptide can be modified by conjugating or fusing it to other polypeptide or polypeptide domains such as, by way of non-limiting example, transferrin (WO06096515A2), albumin (Yeh et al., 1992), growth hormone (US2003104578AA); cellulose (Levy and Shoseyov, 2002); and/or Fc fragments (Ashkenazi and Chamow, 1997). A GDF11 polypeptide as described herein can comprise at least one peptide bond replacement. A single peptide bond or multiple peptide bonds, e.g. 2 bonds, 3 bonds, 4 bonds, 5 bonds, or 6 or more bonds, or all the peptide bonds can be replaced. An isolated peptide as described herein can comprise one type of peptide bond replacement or multiple types of peptide bond replacements, e.g. 2 types, 3 types, 4 types, 5 types, or more types of peptide bond replacements. Nonlimiting examples of peptide bond replacements include urea, thiourea, carbamate, sulfonyl urea, trifluoroethylamine, ortho-(aminoalkyl)-phenylacetic acid, para-(aminoalkyl)-phenylacetic acid, meta (aminoalkyl)-phenylacetic acid, thioamide, tetrazole, boronic ester, olefinic group, and derivatives thereofA GDF11 polypeptide as described herein can comprise naturally occurring amino acids commonly found in polypeptides and/or proteins produced by living organisms, e.g. Ala (A), Val (V), Leu (L), Ile (I), Pro (P), Phe (F), Trp (W), Met (M), Gly (G), Ser (S), Thr (T), Cys (C), Tyr (Y), Asn (N), Gln (Q), Asp (D), Glu (E), Lys (K), Arg (R), and His (H). A GDF11 polypeptide as described herein can comprise alternative amino acids. Non-limiting examples of alternative amino acids include, D-anlino acids; beta-amino acids; homocysteine, phosphoserine, phosphothreonine, phosphotyrosine, hydroxyproline, gamma-carboxyglutamate; hippuric acid, octahydroindole-2-carboxylic acid, statine, 1 ,2,3,4,-tetrahydroisoquinoline-3-carboxylic acid, penicillamine (3-mercapto-D-valine), ornithine, citruline, alphamethyl-alanine, parabenzoylphenylalanine, para-amino phenylalanine, p-fluorophenylalanine, phenylglycine, propargylglycine, sarcosine, and tert-butylglycine), diaminobutyric acid, 7-hydroxytetrahydroisoquinoline carboxylic acid, naphthylalanine, biphenylalanine, cyclohexylalanine, aminoisobutyric acid, norvaline, norleucine, tert-leucine, tetrahydroisoquinoline carboxylic acid, pipecolic acid, phenylglycine, homophenylalanine, cyclohexylglycine, dehydroleucine, 2,2-diethylglycine, 1-amino-1cyclopentanecarboxylic acid, 1-amino-1-cyclohexanecarboxylic acid, amino-benzoic acid, aminonaphthoic acid, gamma-aminobutyric acid, difluorophenylalanine, nipecotic acid, alpha-anlino butyric acid, thienyl-alanine, t-butylglycine, trifluorovaline; hexafluoroleucine; fluorinated analogs; azidemodified amino acids; alkyne-modified amino acids; cyano-modified amino acids; and derivatives thereof.

A GDF11 polypeptide can be modified, e.g. by addition of a moiety to one or more of the amino acids comprising the peptide. A GDF11 polypeptide as described herein can comprise one or more moiety molecules, e.g. 1 or more moiety molecules per peptide, 2 or more moiety molecules per peptide, 5 or more moiety molecules per peptide, 10 or more moiety molecules per peptide or more moiety molecules per peptide. In some embodiments, a GDF11 polypeptide as described herein can comprise one more types of modifications and/or moieties, e.g. 1 type of modification, 2 types of modifications, 3 types of modifications or more types of modifications. Non-limiting examples of modifications and/or moieties include PEGylation; glycosylation; HESylation; ELPylation; lipidation; acetylation; amidation; end-capping modifications; cyano groups; phosphorylation; albumin, and cyclization. In some embodiments, an end-capping modification can comprise acetylation at the N-terminus, N-terminal acylation, and N-terminal formylation. In some embodiments, an end-capping modification can comprise amidation at the C terminus, introduction of C-terminal alcohol, aldehyde, ester, and thioester moieties. The half-life of a GDF11 polypeptide can be increased by the addition of moieties, e.g. PEG or albumin.

The GDF11 polypeptide administered to the subject can be a functional fragment of one of the GDF11 amino acid sequences described herein. As used herein, a "functional fragment" is a fragment or segment of a peptide which can rejuvenate neural stem cells and/or progenitor cells or increase neurogenesis in a subject in accordance with the work described herein. A functional fragment can comprise conservative substitutions of the sequences disclosed herein. A functional fragment can comprise the 12.5 kDa C-terminus of GDF11. The 12.5 kDa C-terminus of GDF11 can function as a monomer. The 12.5 kDa C-terminus of GDF11 can function as a homodimer. The 12.5 kDa C-tenninus of GDF11 can function as a heterodimer with the GDF11 pro-peptide.

Alterations of the original amino acid sequence can be accomplished by any of a number of techniques known to one of skill in the art. Mutations can be introduced, for example, at particular loci by synthesizing oligonucleotides containing a mutant sequence, flanked by restriction sites permitting ligation to fragments of the native sequence. Following ligation, the resulting reconstructed sequence encodes an analog having the desired amino acid insertion, substitution, or deletion. Alternatively, oligonucleotide-directed site-specific mutagenesis procedures can be employed to provide an altered nucleotide sequence having particular codons altered according to the substitution, deletion, or insertion required. Techniques for making such alterations include those disclosed by Walder et al. (Gene 42:133, 1986); Bauer et al. (Gene 37:73, 1985); Craik (BioTechniques, January 1985, 12-19); Smith et al. (Genetic Engineering: Principles and Methods, Plenum Press, 1981); and U.S. Pat. Nos. 4,518,584 and 4,737,462. A GDF11 polypeptide as described herein can be chemically synthesized and mutations can be incorporated as part of the chemical synthesis process.

A GDF11 polypeptide as described herein can be formulated as a pharmaceutically acceptable prodrug. As used herein, a "prodrug" refers to compounds that can be converted via some chemical or physiological process (e.g., enzymatic processes and metabolic hydrolysis) to a therapeutic agent. Thus, the term "prodrug" also refers to a precursor of a biologically active compound that is pharmaceutically acceptable. A prodrug may be inactive when administered to a subject, i.e. an ester, but is converted *in vivo* to an active compound, for example, by hydrolysis to the free carboxylic acid or free hydroxyl. The prodrug compound often offers advantages of solubility, tissue compatibility or delayed release in an organism. The term "prodrug" is also meant to include any covalently bonded carriers, which release the active compound *in vivo* when such prodrug is administered to a subject. Prodrugs of an active compound may be prepared by modifying functional groups present in the active compound in such a way that the modifications are cleaved, either in routine manipulation or *in vivo,* to the parent active compound. Prodrugs include compounds wherein a hydroxy, amino or mercapto group is bonded to any group that, when the prodrug of the active compound is administered to a subject, cleaves to form a free hydroxy, free amino or free mercapto group, respectively. Examples of prodrugs include, but are not limited to, acetate, formate and benzoate derivatives of an alcohol or acetamide, formamide and benzamide derivatives of an amine functional group in the active compound and the like. See Harper, "Drug Latentiation" in Jucker, ed. Progress in Drug Research 4:221-294 (1962); Morozowich et al, "Application of Physical Organic Principles to Prodrug Design" in E. B. Roche ed. Design of Biophamzaceutical Properties through Prodrugs and Analogs, APHA Acad. Pharm. Sci. 40 (1977); Bioreversible Carriers in Drug in Drug Design, Theory and Application, E. B. Roche, ed., APHA Acad. Pharm. Sci. (1987); Design of Prodrugs, H. Bundgaard, Elsevier (1985); Wang et al. "Prodrug approaches to the improved delivery of peptide drug" in Curr. Pharm. Design. 5(4):265-287 (1999); Pauletti et al. (1997) Improvement in peptide bioavailability: Peptidomimetics and Prodrug Strategies, Adv. Drug. Delivery Rev. 27:235-256; Mizen et al. (1998) "The Use of Esters as Prodrugs for Oral Delivery of (3-Lactam antibiotics," Pharm. Biotech. 11,:345-365; Gaignault et al. (1996) "Designing Prodrugs and Bioprecursors I. Carrier Prodrugs," Pract. Med. Chern. 671-696; Asgharnejad, "Improving Oral Drug Transport", in Transport Processes in Pharmaceutical Systems, G. L. Amidon, P. I. Lee and E. M. Topp, Eds., Marcell Dekker, p. 185-218 (2000); Balant et al., "Prodrugs for the improvement of drug absorption via different routes of administration", Eur. J. Drug Metab. Pharmacokinet., 15(2): 143-53 (1990); Balimane and Sinko, "Involvement of multiple transporters in the oral absorption of nucleoside analogues", Adv. Drug Delivel)'Rev., 39(1-3): 183-209 (1999); Browne, "Fosphenytoin (Cerebyx)", Clin. Neuropharmacol. 20(1): 1-12 (1997); Bundgaard, "Bioreversible derivatization of drugs- principle and applicability to improve the therapeutic effects of drugs", Arch. Pharm. Chemi 86(1): 1-39 (1979); Bundgaard H. "Improved drug delivery by the prodrug approach", Controlled Drug Delively 17: 179-96 (1987); Bundgaard H. "Prodrugs as a means to improve the delivery of peptide drugs" ,Arfv. Drug Delivel)' Rev. 8(1): 1-38 (1992); Fleisher et al. "Improved oral drug delivery: solubility limitations overcome by the use of prodrugs", Arfv. Drug Delivery Rev. 19(2): 115-130 (1996); Fleisher et al. "Design of prodrugs for improved gastrointestinal absorption by intestinal enzyme targeting", Methods Enzymol. 112 (Drug Enzyme Targeting, Pt. A): 360-81, (1985); Farquhar D, et al., "Biologically Reversible Phosphate-Protective Groups", Pharm. Sci., 72(3): 324-325 (1983); Freeman S, et al., "Bioreversible Protection for the Phospho Group: Chemical Stability and Bioactivation of Di( 4-acetoxybenzyl) Methylphosphonate with Carboxyesterase," Chern. Soc., Chern. Commun., 875-877 (1991); Friis and Bundgaard, "Prodrugs of phosphates and phosphonates: Novel lipophilic alphaacyloxyalkyl ester derivatives of phosphate- or phosphonate containing drugs masking the negative charges of these groups", Eur. J. Pharm. Sci. 4: 49-59 (1996); Gangwar et al., "Pro-drug, molecular structure and percutaneous delivery", Des. Biophamz. Prop. Prodrugs Analogs, [Symp.] Meeting Date 1976,409-21. (1977); Nathwani and Wood, "Penicillins: a current review of their clinical pharmacology and therapeutic use", Drugs 45(6): 866-94 (1993); Sinhababu and Thakker, "Prodrugs of anticancer agents", Adv. Drug Delivery Rev. 19(2): 241-273 (1996); Stella et al., "Prodrugs. Do they have advantages in clinical practice?", Drugs 29(5): 455-73 (1985); Tan et al. "Development and optimization of anti-HIV nucleoside analogs and prodrugs: A review of their cellular pharmacology, structure-activity relationships and pharmacokinetics", Adv. Drug Deliva}' Rev. 39(1-3): 117-151 (1999); Taylor, "Improved passive oral drug delivery via prodrugs", Adv. Drug Delivery Rev., 19(2): 131-148 (1996); Valentino and Borchardt, "Prodrug strategies to enhance the intestinal absorption of peptides", Drug Discovery Today 2(4): 148-155 (1997); Wiebe and Knaus, "Concepts for the design of anti-HIV nucleoside prodrugs for treating cephalic HIV infection", Adv. Drug Delivery Rev.: 39(1-3):63-80 (1999); Waller et al., "Prodrugs", Br. J. Clin. Pharmac. 28: 497-507 (1989).

A GDF11 polypeptide as described herein can be a pharmaceutically acceptable solvate. The term "solvate" refers to a peptide as described herein in the solid state, wherein molecules of a suitable solvent are incorporated in the crystal lattice. A suitable solvent for therapeutic administration is physiologically tolerable at the dosage administered. Examples of suitable solvents for therapeutic administration are ethanol and water. When water is the solvent, the solvate is referred to as a hydrate. In general, solvates are formed by dissolving the compound in the appropriate solvent and isolating the solvate by cooling or using an antisolvent. The solvate is typically dried or azeotroped under ambient conditions.

The peptides described herein can be synthesized by using well known methods including recombinant methods and chemical synthesis. Recombinant methods of producing a peptide through the introduction of a vector including nucleic acid encoding the peptide into a suitable host cell is well known in the art, such as is described in Sambrook et al., Molecular Cloning: A Laboratory Manual, 2d Ed, Vols 1 to 8, Cold Spring Harbor, NY (1989); M.W. Pennington and B.M. Dunn, Methods in Molecular Biology: Peptide Synthesis Protocols, Vol 35, Humana Press, Totawa, NJ (1994). Peptides can also be chemically synthesized using methods well known in the art. See for example, Merrifield et al., J. Am. Chern. Soc. 85:2149 (1964); Bodanszky, M., Principles of Peptide Synthesis, Springer-Verlag, New York, NY (1984); Kirnrnerlin, T. and Seebach, D. J. Pept. Res. 65:229-260 (2005); Nilsson et al., Annu. Rev. Biophys. Biornol. Struct. (2005) 34:91-118; W.C. Chan and P.D. White (Eds.) Frnoc Solid Phase Peptide Synthesis: A Practical Approach, Oxford University Press, Cary, NC (2000); N.L. Benoiton, Chemistry of Peptide Synthesis, CRC Press, Boca Raton, FL (2005); J. Jones, Amino Acid and Peptide Synthesis, 2nd Ed, Oxford University Press, Cary, NC (2002); and P. Lloyd-Williams, F. Albericio, and E. Giralt, Chemical Approaches to the synthesis of pep tides and proteins, CRC Press, Boca Raton, FL ( 1997). Peptide derivatives can also be prepared as described in U.S. Pat. Nos. 4,612,302; 4,853,371; and 4,684,620, and U.S. Pat. App. Pub. No. 2009/0263843.

The technology described herein relates to a nucleic acid encoding a GDF11 polypeptide as described herein. As used herein, the term "nucleic acid" or "nucleic acid sequence" refers to any molecule, preferably a polymeric molecule, incorporating units of ribonucleic acid, deoxyribonucleic acid or an analog thereof. The nucleic acid can be either single-stranded or double-stranded. A single-stranded nucleic acid can be one strand nucleic acid of a denatured double stranded DNA. Alternatively, it can be a single-stranded nucleic acid not derived from any double stranded DNA. In one aspect, the template nucleic acid is DNA. In another aspect, the template is RNA. Suitable nucleic acid molecules are DNA, including genomic DNA or cDNA. Other suitable nucleic acid molecules are RNA, including mRNA. The nucleic acid molecule can be naturally occurring, as in genomic DNA, or it may be synthetic, i.e., prepared based upon human action, or may be a combination of the two. The nucleic acid molecule can also have certain modification such as 2'-deoxy, 2'-deoxy-2'fluoro, 2'-0-methyl, 2'-0-methoxyethyl (2'-0-MOE), 2'-0-aminopropyl (2'-0-AP), 2'-0-dimethylaminoethyl (2'-0-DMAOE), 2'-0-dimethylaminopropyl (2'-0-DMAP), 2'-0-dimethylaminoethyloxyethyl (2'-0-DMAEOE), or 2'-0--N-methylacetamido (2'-0-NMA), cholesterol addition, and phosphorothioate backbone as described in US Patent Application Publication 2007/0213292; and certain ribonucleoside that are is linked between the 2' -oxygen and the 4' -carbon atoms with a methylene unit as described in US Pat No. 6,268,490.

A nucleic acid encoding a GDF11 polypeptide can comprise the nucleotide sequence of SEQ ID NO: 5 (NCBI Ref Seq: NM_005811.3). A nucleic acid encoding a GDF11 polypeptide as described herein may be comprised by a vector. A nucleic acid sequence encoding a GDF11 polypeptide as described herein, or any module thereof, may be operably linked to a vector. The term "vector," as used herein, refers to a nucleic acid construct designed for delivery to a host cell or for transfer between different host cells. As used herein, a vector can be viral or non-viral. The term "vector" encompasses any genetic element that is capable of replication when associated with the proper control elements and that can transfer gene sequences to cells. A vector can include, but is not limited to, a cloning vector, an expression vector, a plasmid, phage, transposon, cosmid, chromosome, virus, virion, etc.

As used herein, the term "expression vector" refers to a vector that directs expression of an RNA or polypeptide from sequences linked to transcriptional regulatory sequences on the vector. The sequences expressed will often, but not necessarily, be heterologous to the cell. An expression vector may comprise additional elements, for example, the expression vector may have two replication systems, thus allowing it to be maintained in two organisms, for example in human cells for expression and in a prokaryotic host for cloning and amplification. The term "expression" refers to the cellular processes involved in producing RNA and proteins and as appropriate, secreting proteins, including where applicable, but not limited to, for example, transcription, transcript processing, translation and protein folding, modification and processing. "Expression products" include RNA transcribed from a gene, and polypeptides obtained by translation of mRNA transcribed from a gene. The term "gene" means the nucleic acid sequence which is transcribed (DNA) to RNA *in vitro* or *in vivo* when operably linked to appropriate regulatory sequences. The gene may or may not include regions preceding and following the coding region, e.g. 5' untranslated (5'UTR) or "leader" sequences and 3' UTR or "trailer" sequences, as well as intervening sequences (introns) between individual coding segments (exons).

As used herein, the term "viral vector" refers to a nucleic acid vector construct that includes at least one element of viral origin and has the capacity to be packaged into a viral vector particle. The viral vector can contain the nucleic acid encoding a GDF11 polypeptide as described herein in place of non-essential viral genes. The vector and/or particle may be utilized for the purpose of transferring any nucleic acids into cells either *in vitro* or *in vivo.* Numerous forms of viral vectors are known in the art.

By "recombinant vector" is meant a vector that includes a heterologous nucleic acid sequence, or "transgene" that is capable of expression *in vivo.* It should be understood that the vectors described herein can, in some embodiments, be combined with other suitable compositions and therapies. In some embodiments, the vector is episomal. The use of a suitable episomal vector provides a means of maintaining the nucleotide of interest in the subject in high copy number extra chromosomal DNA thereby eliminating potential effects of chromosomal integration.

The level of GDF11 in the subject may be increased by at least 20% over the level of GDF11 in the subject prior to treatment, e.g. 20% or more, 30% or more, 40% or more, 50% or more, 100% or more, 150% or more, 200% or more, 250% or more, 300% or more, or 350% or more. The level of GDF11 in the subject may be increased by at least 100% over the level of GDF11 in the subject prior to treatment. The level of GDF11 in the subject may be increased by at least 200% over the level of GDF11 in the subject prior to treatment. The level of GDF11 in the subject may be increased by about 250% over the level of GDF11 in the subject prior to treatment. The level of GDF11 in the subject may be increased to at least 50% of a healthy reference level, e.g. 50% or more, 60% or more, 70% or more, 80% or more, 90% or more, or 100% or more of a healthy reference level. The level of GDF11 in the subject may be increased to at least 60% of a healthy reference level. The level of GDF11 in the subject may be increased to at least 75% of a healthy reference level. The level of GDF11 in the subject may be increased to at least 90% of a healthy reference level. A healthy reference level can be the average level of GDF11 in a population of human subjects (e.g., young individuals) not exhibiting any signs or symptoms of neural stem cell and/or progenitor cell deterioration, diminished capacity for neurogenesis, or related conditions.

As used herein, "neural stem cell and/or progenitor cell deterioration" refers to a decrease in cell number or frequency of neural stem cells and/or progenitor cells.

A healthy reference level can be the average level of GDF11 in a population of human subjects not exhibiting any signs or symptoms of neural stem cell and/or progenitor deterioration, diminished capacity for neurogenesis or related conditions and who are under the age of 70. A healthy reference level can be the average level of GDF11 in a population of human subjects not exhibiting any signs or symptoms of neural stem cell and/or progenitor deterioration, diminished capacity for neurogenesis, or related conditions and who are under the age of 65. A healthy reference level can be the average level of GDF11 in a population of human subjects not exhibiting any signs or symptoms of neural stem cell and/or progenitor deterioration, diminished capacity for neurogenesis, or related conditions and who are under the age of 60. A healthy reference level can be the average level of GDF11 in a population of human subjects not exhibiting any signs or symptoms of neural stem cell and/or progenitor deterioration, diminished capacity for neurogenesis, or related conditions and who are under the age of 55. A healthy reference level can be the average level of GDF11 in a population of human subjects not exhibiting any signs or symptoms of neural stem cell and/or progenitor deterioration, diminished capacity for neurogenesis, or related conditions and who are under the age of 50. A healthy reference level can be the average level of GDF11 in a population of human subjects not exhibiting any signs or symptoms of neural stem cell and/or progenitor deterioration, diminished capacity for neurogenesis, or related conditions and who are under the age of 45. A healthy reference level can be the average level of GDF11 in a population of human subjects not exhibiting any signs or symptoms of neural stem cell and/or progenitor deterioration, diminished capacity for neurogenesis, or related conditions and who are under the age of 40. A healthy reference level can be the average level of GDF11 in a population of human subjects not exhibiting any signs or symptoms of neural stem cell and/or progenitor deterioration, diminished capacity for neurogenesis, or related conditions and who are under the age of 35. A healthy reference level can be the average level of GDF11 in a population of human subjects not exhibiting any signs or symptoms of neural stem cell and/or progenitor deterioration, diminished capacity for neurogenesis, or related conditions and who are under the age of 30. A healthy reference level can be the average level of GDF11 in a population of human subjects not exhibiting any signs or symptoms of neural stem cell and/or progenitor deterioration, diminished capacity for neurogenesis, or related conditions and who are under the age of 25. A healthy reference level can be the average level of GDF11 in a population of human subjects not exhibiting any signs or symptoms of neural stem cell and/or progenitor deterioration, diminished capacity for neurogenesis, or related conditions and who are under the age of 20.

The methods described herein can comprise selecting a subject with a level of GDF11 which is lower than a healthy reference level and administering a treatment as described herein.

The level of GDF11 in a subject may be increased in order to treat a neurodegenerative disorder (e.g., Alzheimer's disease, dementia, mild cognitive decline, etc.) or neurological injury or damage to the CNS or the PNS associated with physical injury, ischemia, certain medical procedures or exposure to biological or chemic toxins or poisons, tumors, infections, metabolic or nutritional disorders, cognition or mood disorders, and various medical conditions associated with neural damage or destruction described herein. The level of GDF11 in a subject may be increased in order to treat a neurodegenerative disorder associated with decreased neurogenesis. According to the present invention, the level of GDF11 in a subject is increased in order to treat a neurodegenerative disorder by increasing neurogenesis in a subject. The level of GDF11 in a subject may be increased in order to prevent a neurodegenerative disorder or neurological injury or damage to the CNS or the PNS associated with physical injury, ischemia, certain medical procedures or exposure to biological or chemic toxins or poisons, tumors, infections, metabolic or nutritional disorders, cognition or mood disorders, and various medical conditions associated with neural damage or destruction described herein.

The level of GDF11 in a subject may be increased in order to treat a neurovascular disorder (e.g., stroke). The level of GDF11 in a subject may be increased in order to treat a neurovascular disorder associated with decreased angiogenesis. The level of GDF11 in a subject may be increased in order to treat a neurovascular disorder by increasing angiogenesis in a subject. The level of GDF11 in a subject may be increased in order to prevent a neurovascular disorder (e.g., stroke).

Neurodegenerative or neurovascular disorders related to low or decreased GDF11 polypeptide tend to develop with the decrease in GDF11 levels that occur with increasing age. Thus, it is expected that such conditions can be prevented or, at a minimum, delayed, by maintaining GDF11 polypeptide levels at or near the level found in normal, healthy young adults, e.g., by administering a GDF11 polypeptide or a nucleic acid encoding a GDF11 polypeptide with advancing age, but prior to the onset of a neurodegenerative or neurovascular disorder.

Described herein is a method for treating or preventing a disorder associated with diminished capacity for neurogenesis in a subject, comprising administering to the subject an effective amount of an agent or composition which increases the level of GDF11 polypeptide in the subject, thereby increasing neurogenesis in the subject, wherein increasing neurogenesis in the subject treats or prevents the disorder associated with diminished capacity for neurogenesis in the subject.

Described herein is a method for treating or preventing a neurodegenerative disorder in a subject, comprising administering to the subject an effective amount of an agent or composition which increases the level of GDF11 polypeptide in the subject, thereby treating the neurodegenerative disorder in the subject.

Described herein is a method for treating or preventing a neurodegenerative disorder by increasing neurogenesis in a subject, comprising administering to the subject an effective amount of an agent or composition which increases the level of GDF11 polypeptide in the subject, thereby increasing neurogenesis in the subject, wherein increasing neurogenesis in the subject treats or prevents the neurodegenerative disorder in the subject. In the agent or composition for use according to the invention, increasing neurogenesis is associated with increasing neural cell proliferation, increasing neural cell differentiation, increasing the number of neural stem cells, increasing the number of neural progenitor cells, increasing the number of neural precursor cells, increasing expression of at least one synaptic plasticity gene, at least one neuroprotective gene, or at least one neuronal specification gene selected from the group consisting of neuronal PAS domain protein 4 (Npas4) (NCBI Gene ID: 266743), dual specificity phosphatase 1 (Dusp1) (NCBI Gene ID: 1843), heat shock 70kDa protein 8 (Hspa8) (NCBI Gene ID: 3312), BTG family, member 2 (Btg2) (NCBI Gene ID: 7832), activity-regulated cytoskeleton-associated protein (Arc) (NCBI Gene ID: 23237), nuclear receptor subfamily 4, group A, member 1 (Nr4al) (NCBI Gene ID: 3164), cysteine-rich, angiogenic inducer, 61 (Cyr61) (NCBI Gene ID: 3491), FBJ murine osteosarcoma viral oncogene homolog (Fos) (NCBI Gene ID: 2353), jun B proto-oncogene (Junb) (NCBI Gene ID: 3726), Kruppel-like factor 10 (Klf10) (NCBI Gene ID: 7071), nuclear receptor subfamily 4, group A, member 2 (Nr4a2) (NCBI Gene ID: 4929), claudin 1 (Cldn1) (NCBI Gene ID: 9076), early growth response 2 (Egr2) (NCBI Gene ID: 1959), crystalline, mu (Crym) (NCBI Gene ID: 1428), early growth response 1 (Egr1) (NCBI Gene ID: 1958), and polio-like kinase 2 (Plk2) (NCBI Gene ID: 10769), and increasing the number of new neurons

In some embodiments, neurogenesis is increased in the subventricular zone of the lateral ventricles. In some embodiments, neurogenesis is increased in the olfactory bulb. In some embodiments, increased neurogenesis results in improved olfactory behavior.

The neurodegenerative disorder may be one of polyglutamine expansion disorders (e.g., HD, dentatorubropallidoluysian atrophy, Kennedy's disease (also referred to as spinobulbar muscular atrophy), and spinocerebellar ataxia (e.g., type 1, type 2, type 3 (also referred to as Machado-Joseph disease), type 6, type 7, and type 17)), other trinucleotide repeat expansion disorders (e.g., fragile X syndrome, fragile XE mental retardation, Friedreich's ataxia, myotonic dystrophy, spinocerebellar ataxia type 8, and spinocerebellar ataxia type 12), Alexander disease, Alper's disease, Alzheimer disease, amyotrophic lateral sclerosis (ALS), ataxia telangiectasia, Batten disease (also referred to as Spielmeyer-Vogt-Sjogren-Batten disease), Canavan disease, Cockayne syndrome, corticobasal degeneration, Creutzfeldt-Jakob disease, Guillain-Barré syndrome, ischemia stroke, Krabbe disease, kuru, Lewy body dementia, multiple sclerosis, multiple system atrophy, non-Huntingtonian type of Chorea, Parkinson's disease, Pelizaeus-Merzbacher disease, Pick's disease, primary lateral sclerosis, progressive supranuclear palsy, Refsum's disease, Sandhoff disease, Schilder's disease, spinal cord injury, spinal muscular atrophy (SMA), SteeleRichardson-Olszewski disease, and Tabes dorsalis.

The neurodegenerative disorder may comprise a neurological injury or damage to the CNS or the PNS associated with physical injury, ischemia, certain medical procedures or exposure to biological or chemic toxins or poisons, tumors, infections, metabolic or nutritional disorders, cognition or mood disorders, and various medical conditions associated with neural damage or destruction described herein.

Described herein is a method for treating or preventing a neurovascular disorder, comprising administering to the subject an agent or composition which increases the level of GDF11 polypeptide in the subject, thereby treating or preventing the neurovascular disorder in the subject.

Described herein is a method for treating or preventing a neurovascular disorder by increasing angiogenesis in a subject, comprising administering to the subject an agent or composition which increases the level of GDF11 polypeptide in the subject, thereby increasing angiogenesis in the subject, wherein increasing angiogenesis in the subject treats or prevents the neurovascular disorder in the subject. Increasing angiogenesis may comprise increasing cerebrovascular architecture, increasing capillary density, increasing cerebral blood flow, and increasing cerebral vessel sprouting branch points.

The neurovascular disorder may be selected from the group consisting of brain atherothrombosis, brain aneurysms, brain arteriovenous malformations, brain embolism, brain ischemia, for example caused by atherothrombosis, embolism, or hemodynamic abnormalities, cardiac arrest, carotid stenosis, cerebrovascular spasm, headache, intracranial hemorrhage, ischemic stroke, seizure, spinal vascular malformations, reflex neurovascular dystrophy (RND), neurovascular compression disorders such as hemifacial spasms, tinnitus, trigeminal neuralgia, glossopharyngeal neuralgia, stroke, transient ischemic attacks, and vasculitis.

Aspects of the technology described herein relate to agents and which increase levels of GDF11 polypeptide (e.g., compositions comprising a GDF11 polypeptide as described herein or a nucleic acid encoding a GDF11 polypeptide as described herein). In some embodiments, the composition is a pharmaceutical composition. As used herein, the term "pharmaceutical composition" refers to the active agent in combination with a pharmaceutically acceptable carrier commonly used in the pharmaceutical industry. The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

A composition described herein comprising a GDF11 polypeptide or functional fragment or variant thereof can be used for rejuvenating neural stem cells and/or progenitor cells in a subject in need thereof, wherein increased levels of the GDF11 polypeptide or functional fragment or variant thereof in the subject rejuvenate neural stem cells and/or progenitor cells in the subject.

The composition may cause the subject's neural stem cells to increase in frequency or number, thereby rejuvenating the neural stem cells and/or progenitor cells in the subject.

A composition described herein comprising a GDF11 polypeptide or a functional fragment or variant thereof can be used for increasing neurogenesis in a subject in need thereof, wherein increased levels of the GDF11 polypeptide or functional fragment or variant thereof in the subject increases neurogenesis in the subject. Increasing neurogenesis may be associated with neural cell proliferation, increasing neural cell differentiation, increasing the number of neural stem cells, increasing the number of neural progenitor cells, increasing the number of neural precursor cells, increasing expression of at least one synaptic plasticity gene, at least one neuroprotective gene, or at least one neuronal specification gene selected from the group consisting of neuronal PAS domain protein 4 (Npas4) (NCBI Gene ID: 266743), dual specificity phosphatase 1 (Dusp1) (NCBI Gene ID: 1843), heat shock 70kDa protein 8 (Hspa8) (NCBI Gene ID: 3312), BTG family, member 2 (Btg2) (NCBI Gene ID: 7832), activity-regulated cytoskeleton-associated protein (Arc) (NCBI Gene ID: 23237), nuclear receptor subfamily 4, group A, member 1 (Nr4al) (NCBI Gene ID: 3164), cysteine-rich, angiogenic inducer, 61 (Cyr61) (NCBI Gene ID: 3491), FBJ murine osteosarcoma viral oncogene homolog (Fos) (NCBI Gene ID: 2353), jun B proto-oncogene (Junb) (NCBI Gene ID: 3726), Kruppel-like factor 10 (Klf10) (NCBI Gene ID: 7071), nuclear receptor subfamily 4, group A, member 2 (Nr4a2) (NCBI Gene ID: 4929), claudin 1 (Cldnl) (NCBI Gene ID: 9076), early growth response 2 (Egr2) (NCBI Gene ID: 1959), crystalline, mu (Crym) (NCBI Gene ID: 1428), early growth response 1 (Egr1) (NCBI Gene ID: 1958), and polio-like kinase 2 (Plk2) (NCBI Gene ID: 10769), and increasing the number of new neurons.

A composition described herein comprising a GDF11 polypeptide or a functional fragment or variant thereof can be used for treating or preventing a neurodegenerative disorder in a subject in need thereof, wherein increased levels of the GDF11 polypeptide or functional fragment or variant thereof treat or prevent the neurodegenerative disorder.

According to the present invention, a composition comprising a GDF11 polypeptide of SEQ ID NO: 3 is for use in treating or preventing a neurodegenerative disorder by increasing neurogenesis in a subject, wherein increased levels of the GDF11 polypeptide increase neurogenesis in the subject, thereby treating or preventing the neurodegenerative disorder.

In some embodiments, the subject has been diagnosed with a neurodegenerative disorder due to aging.

A composition described herein comprising a GDF11 polypeptide or functional fragment or variant thereof can be used for increasing angiogenesis in a subject in need thereof, wherein increased levels of the GDF11 polypeptide or functional fragment or variant thereof in the subject increase angiogenesis in the subject. Increasing angiogenesis may comprise increasing cerebrovascular architecture, increasing capillary density, increasing cerebral blood flow, and increasing cerebral vessel sprouting branch points.

A composition described herein comprising a GDF11 polypeptide or functional fragment or variant thereof can be used for treating or preventing a neurovascular disorder in a subject in need thereof, wherein increased levels of the GDF11 polypeptide or functional fragment or variant thereof in the subject treat or prevent the neurovascular disorder in the subject.

A composition described herein comprising a GDF11 polypeptide or functional fragment or variant thereof can be used for treating or preventing a neurovascular disorder by increasing angiogenesis in a subject, wherein increased levels of the GDF11 polypeptide or functional fragment or variant thereof in the subject increase angiogenesis in the subject, thereby treating or preventing the neurovascular disorder in the subject.

Described herein is a pharmaceutical composition or kit of parts for use in rejuvenating neural stem cells and/or progenitor cells, increasing neurogenesis, increasing angiogenesis, or in treating or preventing neurodegenerative or neurovascular disorders.

The preparation of a pharmacological composition that contains active ingredients dissolved or dispersed therein is well understood in the art and generally need not be limited based on formulation. Typically such compositions are prepared as injectable either as liquid solutions or suspensions, however, solid forms suitable for solution, or suspension, in liquid prior to use can also be prepared. The preparation can also be emulsified or presented as a liposome composition. The active ingredient can be mixed with excipients which are pharmaceutically acceptable and compatible with the active ingredient and in amounts suitable for use in the therapeutic methods described herein. Suitable excipients are, for example, water, saline, dextrose, glycerol, ethanol or the like and combinations thereof. In addition, if desired, the composition can contain minor amounts of auxiliary substances such as wetting or emulsifying agents, pH buffering agents, and the like which enhance the effectiveness of the active ingredient. The therapeutic composition of the present invention can include pharmaceutically acceptable salts of the components therein. Pharmaceutically acceptable salts include the acid addition salts (formed with the free amino groups of the polypeptide) that are formed with inorganic acids such as, for example, hydrochloric or phosphoric acids, or such organic acids as acetic, tartaric, mandelic, and the like. Salts formed with the free carboxyl groups can also be derived from inorganic bases such as, for example, sodium, potassium, ammonium, calcium or ferric hydroxides, and such organic bases as isopropylamine, trimethylamine, 2-ethylamino ethanol, histidine, procaine, and the like. Physiologically tolerable carriers are well known in the art. Exemplary liquid carriers are sterile aqueous solutions that contain no materials in addition to the active ingredients and water, or contain a buffer such as sodium phosphate at physiological pH value, physiological saline or both, such as phosphate-buffered saline.

Still further, aqueous carriers can contain more than one buffer salt, as well as salts such as sodium and potassium chlorides, dextrose, polyethylene glycol and other solutes. Liquid compositions can also contain liquid phases in addition to and to the exclusion of water. Exemplary of such additional liquid phases are glycerin, vegetable oils such as cottonseed oil, and water-oil emulsions. The amount of an active agent used in the invention that will be effective in the treatment of a particular disorder or condition will depend on the nature of the disorder or condition, and can be determined by standard clinical techniques.

A GDF11 polypeptide or nucleic acid encoding a GDF11 polypeptide as described herein can be administered by controlled- or delayed-release means. Controlled release pharmaceutical products have a common goal of improving drug therapy over that achieved by their non-controlled release counterparts. Ideally, the use of an optimally designed controlled-release preparation in medical treatment is characterized by a minimum of drug substance being employed to cure or control the condition in a minimum amount of time. Advantages of controlled-release formulations include: 1) extended activity of the drug; 2) reduced dosage frequency; 3) increased patient compliance; 4) usage of less total drug; 5) reduction in local or systemic side effects; 6) minimization of drug accumulation; 7) reduction in blood level fluctuations; 8) improvement in efficacy of treatment; 9) reduction of potentiation or loss of drug activity; and 10) improvement in speed of control of diseases or conditions. Kim, Chemg-ju, Controlled-release Dosage Form Design, 2 (Technomic Publishing, Lancaster, Pa.: 2000).

Conventional dosage forms generally provide rapid or immediate drug release from the formulation. Depending on the pharmacology and pharmacokinetics of the drug, use of conventional dosage forms can lead to wide fluctuations in the concentrations of the drug in a patient's blood and other tissues. These fluctuations can impact a number of parameters, such as dose frequency, onset of action, duration of efficacy, maintenance of therapeutic blood levels, toxicity, side effects, and the like.

Advantageously, controlled-release formulations can be used to control a drug's onset of action, duration of action, plasma levels within the therapeutic window, and peak blood levels. In particular, controlled or extended-release dosage forms or formulations can be used to ensure that the maximum effectiveness of a drug is achieved while minimizing potential adverse effects and safety concerns, which can occur both from under-dosing a drug (i.e., going below the minimum therapeutic levels) as well as exceeding the toxicity level for the drug.

Most controlled-release formulations are designed to initially release an amount of drug (active ingredient) that promptly produces the desired therapeutic effect, and gradually and continually release other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled- release of an active ingredient can be stimulated by various conditions including, but not limited to, pH, ionic strength, osmotic pressure, temperature, enzymes, water, and other physiological conditions or compounds.

A variety of known controlled- or extended-release dosage forms, formulations, and devices can be adapted for use with the salts and compositions of the disclosure. Examples include, but are not limited to, those described in U.S. Pat. Nos.: 3,845,770; 3,916,899; 3,536,809; 3,598,123; 4,008,719; 5674,533; 5,059,595; 5,591 ,767; 5,120,548; 5,073,543; 5,639,476; 5,354,556; 5,733,566; and 6,365,185 B. These dosage forms can be used to provide slow or controlled- release of one or more active ingredients using, for example, hydroxypropylmethyl cellulose, other polymer matrices, gels, permeable membranes, osmotic systems (such as OROS® (Alza Corporation, Mountain View, Calif. USA)), or a combination thereof to provide the desired release profile in varying proportions.

In some embodiments, the technology described herein relates to a syringe comprising a therapeutically effective amount of a composition e.g. a pharmaceutical preparation comprising a GDF11 polypeptide as described herein.

As used herein, the phrase "therapeutically effective amount", "effective amount" or "effective dose" refers to an amount that provides a therapeutic or aesthetic benefit in the treatment, prevention, or management of, for example, neurodegenerative or neurovascular disorder, e.g., an amount that provides a statistically significant decrease in at least one symptom, sign, or marker of the neurodegenerative or neurovascular disorder.

Determination of a therapeutically effective amount is well within the capability of those skilled in the art. Generally, a therapeutically effective amount can vary with the subject's history, age, condition, sex, as well as the severity and type of the medical condition in the subject, and administration of other pharmaceutically active agents.

Described herein is a method comprising administering a GDF11 polypeptide or a nucleic acid encoding a GDF11 polypeptide to a subject. The subject may be in need of treatment for a neurodegenerative disorder, or a related condition as described herein. The subject may be in need of treatment for a neurovascular disorder, or a related condition as described herein. The method may be a method of treating a subject. The method may be a method of treating or preventing sarcopenia in a subject. Such conditions are described herein.

As used herein, "treat," "treatment," "treating," or "amelioration" when used in reference to a disease, disorder or medical condition, refer to therapeutic treatments for a condition, wherein the object is to reverse, alleviate, ameliorate, inhibit, slow down or stop the progression or severity of a symptom or condition. The term "treating" includes reducing or alleviating at least one adverse effect or symptom of a condition. Treatment is generally "effective" if one or more symptoms or clinical markers are reduced. Alternatively, treatment is "effective" if the progression of a condition is reduced or halted. That is, "treatment" includes not just the improvement of symptoms or markers, but also a cessation or at least slowing of progress or worsening of symptoms that would be expected in the absence of treatment. Beneficial or desired clinical results include, but are not limited to, alleviation of one or more symptom(s), diminishment of extent of the deficit, stabilized (i.e., not worsening) state of, for example, dementia, delay or slowing of dementia, and an increased lifespan as compared to that expected in the absence of treatment.

As used herein, the term "administering," refers to the placement of the composition comprising a GDF11 polypeptide or a nucleic acid encoding a GDF11 polypeptide as disclosed herein into a subject by a method or route which results in delivery to a site of action. The pharmaceutical composition comprising a GDF11 polypeptide or a nucleic acid encoding a GDF11 polypeptide can be administered by any appropriate route which results in an effective treatment in the subject.

Data described herein indicate that systemic administration via the vascular system can be effective. Thus administration via the intravenous route is specifically contemplated. However, with appropriate formulation, other routes are contemplated, including, for example, intranasally, intraarterially; intra-coronary arterially; orally, by inhalation, intraperitoneally, intramuscularly, subcutaneously, intracavity, or by other means known by those skilled in the art. The compositions are administered in a manner compatible with the dosage formulation, and in a therapeutically effective amount. The quantity to be administered and timing depends on the subject to be treated, capacity of the subject's system to utilize the active ingredient, and degree of therapeutic effect desired.

Therapeutic compositions containing at least one agent can be conventionally administered in a unit dose, for example. The term "unit dose" when used in reference to a therapeutic composition refers to physically discrete units suitable as unitary dosage for the subject, each unit containing a predetermined quantity of active material calculated to produce the desired therapeutic effect in association with the required physiologically acceptable diluent, i.e., carrier, or vehicle.

The dosage ranges for the agent depends upon the potency, and are amounts large enough to produce the desired effect e.g., rejuvenation of a subject's neural stem cells and/or progenitor cells, increased neurogenesis or angiogenesis, or a reversal of a neurodegenerative disorder or neurovascular disorder. The dosage should not be so large as to cause unacceptable adverse side effects.

Generally, the dosage will vary with the age, condition, and sex of the patient and can be determined by one of skill in the art. The dosage can also be adjusted by the individual physician in the event of any complication. Typically, the dosage can range from 0.001 mg/kg body weight to 0.5 mg/kg body weight. In one embodiment, the dose range is from 5 µg/kg body weight to 30 µg/kg body weight.

Administration of the doses recited above can be repeated. The doses may be given once a day, or multiple times a day, for example, but not limited to, three times a day. The doses recited above may be administered daily for weeks or months. The duration of treatment depends upon the subject's clinical progress and responsiveness to therapy. Without wishing to be bound by theory, where the GDF11 polypeptide apparently diminishes with age in affected individuals, it is expected that long-term therapy would be required to establish and maintain the benefit of GDF11-based treatment, e.g. a neurodegenerative or neurovascular disorder.

Precise amounts of active ingredient required to be administered depend on the judgment of the practitioner and are particular to each individual. However, suitable dosage ranges for systemic application are disclosed herein and depend on the route of administration. Suitable regimes for administration are also variable, but are typified by an initial administration followed by repeated doses at one or more intervals by a subsequent administration. Alternatively, continuous intravenous infusion sufficient to maintain concentrations in the blood in the ranges specified for *in vivo* therapies are contemplated. The dosage range may be sufficient to maintain concentrations in the blood in the range found in the blood of a population of normal, healthy human subjects (e.g. those with no signs, symptoms, or markers of neural stem cell and/or progenitor cell deterioration) under the age of 50. The dosage range may be sufficient to maintain concentrations in the blood in the range found in normal, healthy human subjects under the age of 40. The dosage range may be sufficient to maintain concentrations in the blood in the range found in normal, healthy human subjects under the age of 30. The dosage range may be sufficient to maintain concentrations in the blood in the range found in normal, healthy human subjects under the age of 25. The dosage range may be sufficient to maintain concentrations in the blood in the range found in normal, healthy human subjects under the age of 21.

A therapeutically effective amount is an amount of an agent that is sufficient to produce a statistically significant, measurable change in, for example, a neurodegenerative disorder (e.g., dementia) or neurovascular disorder (e.g., stroke). Such effective amounts can be gauged in clinical trials as well as animal studies. Efficacy of an agent can be determined by assessing physical indicators of, for example neural stem cell and/or progenitor cell deterioration as described above herein. In experimental systems, assays for efficacy include assessing proliferation of neural cells using confocal imaging of SVZ stained by GFAP and Ki67⁺, Olig2⁺, and Sox2⁺, assessing differentiation of neural cells using neurosphere differentiation assays, assessing new neuron formation, for example, by dual staining for BrdU/NeuN⁺, assessing olfactory behavior using an olfaction assay, measuring expression of at least one of the synaptic plasticity, neuroprotective, and neuronal specification genes to assess up-regulation of these genes via microarray or RT-PCR, assessing cerebral blood flow by imaging the vasculature of the SVZ to produce an angiogram for volumetric analysis, and assessing vessel sprouting branch points. Such assays are well known in the art and described in detail in the Examples herein. Clinically acceptable methods for detecting or monitoring neural stem cell rejuvenation are described herein. In addition, efficacy of an agent can be measured by an increase in GDF11 polypeptides or fragments thereof in a subject being treated with an agent comprising a GDF11 polypeptide or a nucleic acid encoding GDF11 polypeptide.

The efficacy of a given treatment for a neurodegenerative or neurovascular disorder can be determined by the skilled clinician. However, a treatment is considered "effective treatment," as the term is used herein, if any one or all of the signs or symptoms of e.g., a neurodegenerative or neurovascular disorder are altered in a beneficial manner, other clinically accepted symptoms are improved or ameliorated, e.g., by at least 10% following treatment with an agent or composition as described herein. Efficacy can also be measured by a failure of an individual to worsen as assessed by hospitalization or need for medical interventions (i.e., progression of the disease is halted). Methods of measuring these indicators are known to those of skill in the art and/or described herein.

The methods described herein may further comprise administering the pharmaceutical composition described herein along with one or more additional agents, biologics, drugs, or treatments beneficial to a subject suffering from neural stem cell and/or progenitor cell deterioration or disorder associated with decreased neurogenesis or angiogenesis, or a neurodegenerative or neurovascular disorder as part of a combinatorial therapy. The agent, biologic, drug, or treatment can be selected from those found in Harrison's Principles of Internal Medicine, 13th Edition, Eds. T.R. Harrison et al. McGraw-Hill N.Y., NY; Physicians Desk Reference, 50th Edition, 1997, Oradell New Jersey, Medical Economics Co.; Pharmacological Basis of Therapeutics, 8th Edition, Goodman and Gilman, 1990; United States Pharmacopeia, The National Formulary, USP XII NF XVII, 1990. The additional pharmaceutically active agent may be selected from the group consisting of butyrates, valproic acid, hydroxyuirae and Riluzole.

The above other therapeutic agents, when employed in combination with the chemical entities described herein, may be used, for example, in those amounts indicated in the Physicians' Desk Reference (PDR) or as otherwise determined by one of ordinary skill in the art.

The details of the description and the examples herein are representative of certain embodiments, are exemplary, and are not intended as limitations on the scope of the invention. Modifications therein encompassed within the scope of the appended claims will occur to those skilled in the art. The articles "a" and "an" as used herein in the specification and in the claims, unless clearly indicated to the contrary, should be understood to include the plural referents. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention also includes embodiments in which more than one, or all of the group members are present in, employed in, or otherwise relevant to a given product or process. Where elements are presented as lists, e.g., in Markush group or similar format, it is to be understood that each subgroup of the elements is also disclosed, and any element(s) can be removed from the group. It should be understood that, in general, where the invention, or aspects of the invention, is/are referred to as comprising particular elements, features, etc., certain embodiments of the invention or aspects of the invention consist, or consist essentially of, such elements, features, etc.

"Approximately" or "about" generally includes numbers that fall within a range of 1% or in some embodiments within a range of 5% of a number or within a range of 10% of a number in either direction (greater than or less than the number) unless otherwise stated or otherwise evident from the context (except where such number would impermissibly exceed 100% of a possible value). It should be understood that unless otherwise indicated or evident from the context, any product or composition described herein may be considered "isolated".

This invention is further illustrated by the following examples which should not be construed as limiting.
***

### EXAMPLES

The examples that follow demonstrate that neurogenesis and angiogenesis in the aged brain can be rejuvenated by heterochronic parabiosis and by GDF11 administration.

Neurogenesis in the adult brain occurs primarily in two areas: the dentate gyrus of the hippocampus and the subventricular zone (SVZ) of the lateral ventricles (*1*, *2*). The capacity of these areas to generate new neurons throughout life lies in the existence of neural stem cells (NSCs) and progenitors. In the SVZ, these cells reside in a three-dimensional heterogeneous niche connected to cerebrospinal fluid and the vasculature. The niche creates a special microenvironment for providing the necessary regulatory cues for proliferation and differentiation (*3-6*). The vasculature has emerged as an important regulator of neural stem cell proliferation and differentiation by providing both factors secreted locally from endothelial cells, as well as systemic regulatory signals from the blood. Systemic regulatory influences include the hormone prolactin (*10*)*,* dietary restriction (*11*) and exercise/enriched environment (*12*), all of which positively modulate neurogenesis, and factors like increased the levels of glucocorticoids associated with stress that have the opposite effect (*13*). In aging, different components of the niche deteriorate. On one hand, the vasculature deteriorates with consequent reduction in blood flow and, on the other hand, the prevalence of NSCs and their capacity to produce new neurons greatly declines with age. This is associated with reduced neuroplasticity and cognitive functioning (*14, 15*). Since the neurovascular niche can respond to systemic signals, it follows that the age-related decline in neurogenesis may reflect alterations in the level of circulating factors, making it also possible to imagine reversing the decline by supplementing or inhibiting these factors. To investigate this concept, the inventors exploited the heterochronic parabiosis paradigm, whereby mice of different ages share a common blood circulation. The work described herein reports for the first time that heterochronic parabiosis resulted in a striking remodeling of the aged cerebral vasculature in response to young systemic factors, producing increased stem and progenitor cell numbers in the SVZ, notable increased angiogenesis and blood flow as well. Furthermore, the neural progenitors are capable of forming new neurons in the olfactory bub (OB), which results in an overall improvement in olfactory behavior. Finally the inventors demonstrate that GDF11 is a systemic factor that recapitulates the beneficial effects of heterochronic parabiosis on angiogenesis and neurogenesis in the old mouse. These studies described herein expand our understanding of age-related decline of neurogenesis to include a direct impact on the neurogenic niche, and further demonstrate that age-dependent remodeling of this niche is reversible by systemic intervention and determinative of neurogenic function.

### Example 1 - Young blood stimulates neural stem and progenitor cell proliferation and expansion in the subventricular zone (SVZ).

Heterochronic parabiotic pairs between old (Het-O) and young (Het-Y) male mice were generated, as well as control groups of age-matched pairs isochronic young (Iso-Y) and isochronic old (Iso-O) **(****Fig.1A****).** All parabiotic pairs remained surgically joined for 5 weeks prior to analysis. To allow identification of circulating cells after parabiosis, young mice carrying the congenic marker CD45.1 and old mice carrying the congenic marker CD45.2 in the heterochronic pair were used, and young mice carrying either CD45.1 or CD45.2 in the Iso-Y pair were used **(****Fig. 5****)**(*20*)*.* By taking advantage of these markers the degree of cross-circulation was assessed by analyzing spleen chimerism after parabiosis **(****Fig. 5****)** which reached around 60%. Coronal sections of the SVZ of heterochronic brains, and their isochronic controls were analyzed first. It was observed that the SVZ area marked by GFAP⁺ cells in the Het-O mice had significantly expanded compared to the same area in the Iso-O mice, suggesting an increase in the proliferative cell population. Indeed, confocal imaging of these sections stained with GFAP in combination with Ki67, Sox2, or Olig2 showed a marked increase in Ki67⁺, Sox2⁺ and Olig2⁺ cell populations **(****Figs. 1B, 1C** and **1D****,** respectively). Quantification of these sections revealed that Ki67⁺ cells increased by 26.9% in the Het-O compared to the Iso-O SVZ **(****Fig. 1E****).** Further analysis of different SVZ stem/progenitor cell types revealed that the number of Sox2⁺ neural stem cells was increased by 112% in the Het-O compared to the Iso-O SVZ **(****Fig. 1F****).** Moreover, Olig2⁺ transit amplifying progenitors in the SVZ area were increased by 57% **(****Fig. 1G****).** On the contrary, these proliferative neural stem/progenitor cell populations remained unaffected by parabiosis in young Het-Y mice **(****Figs. 1H, 1I** and **1J****).**

It has been previously reported that factors in old blood can have detrimental effects on hippocampal neurogenesis in young animals (*31*); however, as described above, the present investigators observed no decrease in neural stem/progenitor cell number in the SVZ in 2-month old young mice joined to 15-month old partners. In an effort to determine whether this discrepancy related to differences between the SVZ and the hippocampus or to the fact that the old mice were younger than the old mice used in the previous study (21 months of age), the present investigators joined 2-month old mice with 21-month old mice, as illustrated in **Fig. 15A****.** In this system, the older blood did negatively affect young SVZ neurogenesis, as Het-Y₂₁ mice showed decreased proliferative Ki67⁺ cells **(****Figs. 15B** and **15D****)** and Sox2⁺ neural stem cell populations **(****Figs. 15C** and **15E****)** in the SVZ. These data are consistent with the previously reported negative effect of older blood on hippocampal neurogenesis (*10*) and indicate an age-dependent accumulation of factors in the blood of older mice that affect neurogenic zones in both the hippocampus and SVZ.

To further examine the effect of heterochronic parabiosis on NSC self-renewal and differentiation potential, neural stem cells from the SVZ area of parabiotic mouse brains were isolated and cultured *in vitro* (*21*)Neural stem cells derived from the SVZ area of parabiotic mouse brains were cultured *in vitro* in the form of neurospheres. After the first passage, neurospheres derived from the Het-O SVZ were 43% larger in diameter than those derived from the Iso-O SVZ **(****Fig. 6****),** suggesting that the proliferation rate was higher, and that NSC retain their ability to proliferate more, even *ex vivo,* once they have been exposed to young systemic factors, which is in accordance with the *in vivo* data. To assess if NSC differentiation was affected as well, a neurosphere differentiation assay was performed by removing the growth factors in culture (22). Indeed, Het-O differentiated neurospheres generated approximately 2.5-fold more TuJ1⁺ neurons than the Iso-O (**Figs. 7A, 7B,** and **7C****),** confirming that the rejuvenation effects of heterochronic parabiosis extend to promote neural progenitor cell differentiation *in vitro.* Collectively these data demonstrate that youthful circulating factors can restore the self-renewal and differentiation potential of aged stem cells and progenitors and that this effect can persist for some time after isolation from the mouse brain.

### Example 2 - Heterochronic parabiosis enhances neurogenesis and cognitive functions in the old mouse.

Adult subventricular zone (SVZ) neural stem and progenitor cells differentiate into neuroblasts and migrate through the rostral migratory stream in order to finally reach the olfactory bulb where they mature locally into interneurons (23). The inventors next hypothesized that the increase in neural stem cells (NSCs) and progenitor cells reflects a concurrent change in olfactory neurogenesis. Parabiotic pairs were injected with BrdU (50mg/kg) one week after joining and pulsed every 12 hours for 3 days in order to label newborn neurons. After a total of 5 weeks, the animals were analyzed for double labeled BrdU⁺ and NeuN⁺ cells to quantify newborn neurons **(****Fig. 2A****).** Interestingly, increased self-renewal and differentiation potential of NSCs and progenitor cells resulted in increased neurogenesis in the olfactory bulb. Het-O newborn neuron populations were enriched by 92% compared to the Iso-O **(****Fig. 2B****).** Neurons in the Het-Y condition seemed to be slightly negatively affected, although the decrease in neurogenesis was not statistically significant **(****Fig. 2C****).**

To further test the functional significance of these findings an olfaction assay was performed where naive parabionts separated from the parabiotic partners after 5 weeks of joining were exposed to an odorant **(****Fig. 8****)** (*24*). After a short habituation period, each parabiont was presented with different concentrations of an odorant, and the total time that each parabiont spent exploring the odorant for each concentration was measured. In this assay, Het-O mice spent more time exploring the odorant at low concentration (10⁻⁵), whereas high concentrations (10⁻¹) of the odorant seemed to produce a negative response **(****Fig. 2D****).** The same was true for young controls **(****Fig. 2D****).** On the other hand, Iso-O mice spent roughly the same amount of time exploring the odorant regardless of its concentration **(****Fig. 2D****).** To ensure that these results were not due to habituation to the odorant, the inventors performed the test starting from both lower and higher concentrations. In this paradigm Het-O mice showed an improved olfactory capability and higher olfactory sensitivity than the Iso-O mice, since they discriminate better between different concentrations of the odorant and needed far less time to discover the odorant compared to Iso-O pairs **(****Fig. 2D****).** Therefore, heterochronic parabiosis and exposure of the neurogenic niche to young systemic factors enhances functional neurogenesis culminating in an improvement in olfactory behavior.

This observation encouraged the inventors to explore the molecular changes that could be associated with the above functional modifications after parabiosis. A microarray analysis on the forebrain of isochronic and heterochronic mice was performed. Gene expression changes were compared between all different conditions for both up-regulation and down-regulation, resulting in multiple Venn diagrams **(****Figs. 9A** and **9B****).** Strikingly, the cluster of genes that were up-regulated by up to 16-fold in both Iso-Y and Het-O conditions compared with Iso-O condition contains almost exclusively activity-dependent genes, largely known for their roles in synaptic plasticity, neuroprotection and neuron specification **(****Fig. 2E****).** Examples of highly up-regulated genes include Nasp4, Fos, Klf10, Dusp1, Btg2 and Arc. Npas4 is an immediate early gene that regulates the expression of activity-dependent genes (25). These results were further confirmed by real-time RT-PCR in which the inventors used different parabiotic animals than the ones used for the microarray analysis. Genes related to neuronal activity, like Npas4, Duspl, Btg2, Nra1 and Cyr61 were up-regulated in the Het-O mice compared to the Iso-O mice **(****Fig. 2F****).** These data correlate with the above findings and provide additional evidence for the role of heterochronic parabiosis in the enhancement of neuroplasticity.

### Example 3 - Young blood enhances vascularization and increases blood flow in the old mouse.

Since neural stem cells (NSCs) and the vasculature are functionally linked in the subventricular zone (SVZ) niche (4-6, 26), the inventors investigated whether parabiosis could affect the aged vasculature. Cerebrovascular architecture, capillary density and cerebral blood flow have been reported to decline during aging *(17-19).* The inventors reasoned that increases in neurogenesis in the aged brains might be due to a restoration of blood vessel number and function. For this purpose the vasculature of the SVZ was imaged and 3D reconstructions of the blood vessels or "angiograms" were created **(****Fig. 10A****).** Volumetric analysis of these angiograms showed that aging causes a decrease in blood vessel volume (comparison between a young and an old mouse) **(****Figs. 3A** and **3B****).** However, heterochronic parabiosis reversed this decline, enhancing angiogenesis and increasing blood vessel volume by 87% in the Het-O compared to the Iso-O group **(****Fig. 3A** and **3C****).** Further quantification of vessel sprouting branch points showed a 21% increase in Het-O versus Iso-O mice **(****Fig. 3D****).** Moreover, some of the blood vessels in the Het-O mice were not associated with AQP4⁺ astrocytic endfeet, suggesting that these vessels are newly formed and potentially leaky thus providing NSCs with enhanced nutritive support **(****Fig. 10B****).** This phenomenon of increased vascularization in the Het-O mice extended to other neurogenic areas like the hippocampus **(****Figs. 16A** and **16B****)** and also to non-neurogenic areas in the brain, like the cortex **(****Fig. 10C****).** Young vasculature (Iso-Y and Het-Y), to the contrary, retained the same volumetric and branching characteristics despite heterochronic parabiosis **(****Fig. 3A, 3E,** and **3F****).**

Because new vessels can form by either spontaneous sprouting from existing capillaries or *de novo* from circulating endothelial progenitors or both, the inventors asked which mechanism may be responsible for the increased volume of the vasculature that the inventors observed in heterochronic parabiosis. To determine the contribution of circulating progenitors, young GFP mice were joined with old non-GFP mice for 5 weeks. Analysis of these parabiotic brains demonstrated that endothelial progenitors from the young mice do not contribute substantially to the vascular remodeling in the Het-O mice **(****Fig. 11****),** reinforcing the idea that angiogenesis is stimulated by circulating factors. This conclusion was further supported when primary brain capillary endothelial cells were cultured *in vitro* and treated with serum isolated from either a young or an old mouse. As pericytes play a role in vasoconstriction in capillaries, the present inventors next sought to investigate whether their numbers were altered by heterochronic parabiosis. As illustrated in **Fig. 17****,** quantification of the number of pericytes associated with blood vessels showed that they were unaffected by parabiosis. Young serum stimulated endothelial cell proliferation by 1.8-fold compared to old serum **(****Fig. 12****),** correlating with our *in vivo* data and indicating that the age of the mouse from which serum was isolated, rather than the age of the cells themselves, is critical to the stimulation of proliferation.

Next the inventors asked whether the increased blood vessel volume would translate into any type of functional improvement or modification. Since cerebral blood flow (CBF) is known to decrease with aging (27), CBF was measured by magnetic resonance imaging (MRI) in the SVZ of parabiotic mice **(****Fig. 3G****).** Indeed, CBF decreased with aging, as Iso-O mice had lower CBF than Iso-Y mice **(****Fig. 3H****).** However, when CBF was measured in Het-O mice it was noticeably higher than in Iso-O mice and close to the young levels **(****Fig. 3G** and **3I****)** suggesting that the induction of angiogenesis observed in the Het-O mouse results in a change in the hemodynamics of the vascular system.

### Example 4 - Young circulating factor GDF11 recapitulates the effect of heterochronic parabiosis by enhancing angiogenesis and neurogenesis.

The above data suggest that circulating factors in the young blood stimulate angiogenesis and neurogenesis in the older brain. This prompted the inventors to investigate whether GDF11, a circulating factor, could restore the age-related decline in angiogenesis and neurogenesis. For that reason, aged mice were treated with daily injections of either recombinant GDF11 (rGDF1 1, 0.1mg/kg mouse body weight, a dosing regimen that increases GDF11 levels in old mice toward youthful levels) or PBS (vehicle) for 4 weeks and their blood vessels were analyzed using the volumetric assay described above. Surprisingly, the volume of blood vessels in GDF11-treated old mice increased by 50% compared to the PBS-treated mice **(****Figs. 4A** and **4B****).** Moreover, the population of Sox2⁺ cells in GDF11-treated old mice increased by 29% compared to the control **(****Figs. 4C** and **4D****).** *In vitro* experiments confirmed that a 6-day treatment of primary brain capillary endothelial cells with rGDFl 1 (40ng/ml) *in vitro* increased their proliferation by 1.23-fold compared to controls, suggesting that GDF11 can act directly on endothelial cells **(****Fig.4E** and **4F****).** GDF11 is a member of the TGFβ superfamily and its receptor is ALK4/5. Activation of the receptor should lead to phosphorylation of SMAD2/3 proteins as the first component of the signaling cascade. Indeed, treating endothelial cells with either rGDF11 (40ng/ml) or TGFβ (positive control, 10ng/ml) increased SMAD phosphorylation significantly, although GDF11 was somewhat less active than TGFβ under these conditions. Thus, GDF11 promotes proliferation of brain capillary endothelial cells through the SMAD signaling cascade **(****Figs. 18A** and **18B****).**

### Conclusions

The work described herein demonstrates that heterochronic parabiosis induces rejuvenation of the neurogenic niche at least in part by improving vascularity and blood flow, and that the age-related decline in neurogenesis can be reversed by exposure to young circulating factors. The work described herein further shows that the improved neurogenesis in the aged SVZ is associated with an increased number of capillaries in that region. The role of blood vessels in the brain is now believed to be more significant than that of simply providing oxygen and nutrient supply. Previous work has demonstrated a close association between neural stem cells and endothelial cells in the SVZ (4-6). Thus, it is possible that the primary effect of parabiosis is on blood vessels in that area, with an important consequence being the stimulation of NSC proliferation and differentiation. Importantly, the effect of parabiosis on blood vessel number was not restricted to the SVZ, and it may be that additional positive effects of increasing blood flow in the older brain can be observed.

Using one set of dosing conditions, GDF11, recently identified as an important parabiotic factor, was able to increase both angiogenesis and neurogenesis, although not as much as parabiosis itself. It is possible that more optimized dosing will produce greater effects, but it is also possible that there are additional circulating or niche factors that remain to be identified. In any case, it is remarkable that one circulating factor can have such a significant effect and it lends credence to the idea that degenerative conditions of aging are amenable to relatively simple treatments.

### MATERIALS AND METHODS

The materials and methods below were used to carry out the experiments described in the above Examples.

### Animals

Aged C57B1/6 mice (15-17 months) were obtained from the National Institute on Aging (NIA). Young C57B1/6J, B6.SJL and GFP mice (2 months) were obtained from Jackson Laboratories (USA). All animal studies were approved by Harvard University Institutional Animal Care and Use Committee and performed in accordance with institutional and federal guidelines.

### Parabiosis and blood chimerism analysis

Parabiosis was performed as described previously (28) on young (2 months) and old (15-16 months) male mice. Experiments where older (21 months) mice were used are coded as Iso-O₂₁ and Het-O21. Het-Y₂₁ indicates a Het-Y animal in parabiosis with an Iso-O₂₁ mouse. For reversal of parabiosis mice were anesthetized with isoflurane. The skin was incised at the joining wound, separating the animals. The resulting incisions in the corresponding lateral aspects of separated mice were closed by staples or interrupting suture (5-0 coated vicryl) and animals were given buprenorphine every 12 hours for at least 48 hours after surgery.

For blood chimerism analysis spleens were dissected from the mice, the tissue was homogenized and erythrocytes lysed. Cells were stained with an antibody cocktail (CD45.2-FITC, CD45.1-PE, Terr119-APC, PI). The frequency of chimerism was measured by flow cytometry and analyzed using FLOWJO software (TreeStar, USA). Since old CD45.1⁺ are not commercially available we only used CD45.2⁺ in the Iso-O pair assuming that cross-circulation rates are similar to the Iso-Y and Het pairs.

### Immunohistochemistry

Mice were perfused transcardially with 50ml PBS, followed by 50ml of 4% paraformaldehyde (PFA) and their brains were removed and post-fixed overnight in 4% PFA. Each brain was embedded in 4% agarose and 100µm-thick coronal were cut in a vibrating microtome (VT1000S, Leica) and pre-incubated in 10% normal goat or donkey serum, Triton 0.1% in PBS for 1 hour. For BrdU detection, sections were incubated for 40min with HC1 2N prior to blocking. Tissue sections or cell cultures were incubated overnight at 4°C with the following antibodies: rat monoclonal anti-BrdU (Abcam), mouse monoclonal anti-Olig2 (Millipore), polyclonal rabbit anti-Sox2 (Cell Signaling Technology), polyclonal rabbit anti-ki67 (Abcam), rat monoclonal CD31(BD Biosciences), chicken polyclonal anti-GFAP (Abcam), mouse monoclonal anti-NeuN (Millipore), mouse monoclonal anti-TuJ1 (Millipore), rabbit polyclonal phospho-Smad2/3 (Cell Signaling), rabbit polyclonal anti-Aquaporin 4 and Fluorescein Lycopersicon Esculentum Lectin-FITC (Vector Laboratories). For endothelial proliferation assays the Click-iT® EdU Alexa Fluor® 488 Imaging Kit was used according to the manufacturer's instructions.

### Microarrays

Brains from parabiotic old mice were dissected around the SVZ area and the tissue was processed for RNA extraction which was performed according to the manufacturer's instruction using the Stratagene Absolute RNA Microisolation kit (Stratagene, cat# 400805) and quality-checked on the Agilent 2100 Bioanalyzer. Complementary DNA was prepared with the Ambion WT Expression Kit (Ambion) and quality-checked on the Agilent 2100 Bioanalyzer. The Affymetric GeneChip WT Terminal Labeling Kit was used for cDNA fragmentation, labeling and hybridization to the GeneChip mouse Gene 1.0 ST Array (Affymetrix). The arrays were washed, stained, and scanned using the GeneChip Fluidics Station 450 and GeneChip Scanner 3000 7G (Affymetrix). The obtained raw intensity, .CEL files from the 8 chips, two replicates for each of the four, were then normalized by robust multichip analysis (RMA) (Bioconductor release 2.11). Normalized expression values were analyzed with the Bioconductor limma package, an approach of implementation of the Empirical Bayes linear modeling (Smyth 2005). For all comparison tests, genes with P value <5% were selected for further analyses. The likelihood of overrepresentation of function categories in the up- or down-regulated gene list relative to a background of all array genes was calculated by Fisher's exact test for statistical analysis.

### Neural stem cell cultures, proliferation and differentiation

Neural stem cell cultures (neurospheres) were performed as described previously (22). For proliferation neurospheres were maintained in serum-free medium containing EGF and bFGF (both 20ng/ml). For the differentiation assay neurospheres were trypsinized and plated on poly-lysine/laminin coated coverslips in serum-free, growth-factor-free medium as described previously (22).

### Endothelial cell cultures

Primary mouse brain microvascular endothelial cells were purchased from Cell Biologies and maintained in Endothelial Cell Medium according to vendor's instructions. For proliferation assays, BVECs were trypsinized and seeded in a gelatin-coated 96 well plate at a density of 2,000 cells/well. Complete medium was replaced with the experimental growth medium 12 hours after plating. EdU was added at a concentration of 10µM four hours prior to fixation with 4% paraformaldehyde. Cells were stained with the EdU kit and the nuclear dye Hoechst 33342. For pSMAD assays, primary brain vessel endothelial cells were treated as indicated, in the presence of sodium orthovanadate, to preserve phosphorylation for 30 minutes, fixed with 4% paraformaldehyde, subjected to immunocytochemical analysis, and imaged using the Operetta high content imaging microsope (Perkin Elmer). Automated image analysis was performed using Columbus software (Perkin Elmer).

### Olfactory sensitivity assay

This test is designed to detect a difference in olfactory sensitivity between two groups of mice. The olfactory sensitivity assay was performed as previously described (*24*). Before the assay control mice were exposed to different sets of odorants in order to measure the spontaneous response to the different odorants and concentrations. For the assay every naive parabiotic mouse (3 days after pair separation) was placed in a new, clean cage and left for 15 min for habituation. Then different dilutions of the odorant (Peppermint, Sigma-Aldrich) that ranged from 10 to 10,000 were infused on a disk and the disk placed in the cage across form the mouse. Different doses of the odorant were presented to the animal. To avoid bias, the experiment was conducted both from high to low dilution and the reverse. The total time of exploration was measured and data were analyzed by Graph Pad Prism.

### Perfusion MRI

Relative cerebral blood flow (CBF) was measured by using a flow-sensitive alternating inversion recovery (FAIR) sequence (29) on a Bruker Pharmascan 4.7T MRI using endogenous water protons as a tracer (TR = 16000 ms, TE = 23 ms, 64 x 64 x 1 matrix size, 0.625 mm x 0.625 mm x 2 mm voxel size) and with a Bruker 3.7 cm diameter transmit-receive coil to fit both mice in the scanner. This arterial spin labeling technique performs two echo planar images (EPI) with both flow sensitive and flow insensitive EPI scans. During the inversion delay time after slice-selective inversion, fully magnetized blood protons move into the imaging slice and exchange with tissue water protons. The difference between these two images is directly proportional to relative blood flow. In addition, a T2-weighted (T2w) scan (TR = 2000 ms, TE = 20 ms, effective TE = 60 ms, 8 averages, 192 x 192 x 22 matrix size, 0.133 mm x 0.130 mm x 0.7 mm voxel size) was performed to select a slice in the subventricular zone. The geometry was customized for each of the parabiosed mice to ensure axial slice selection for the images. The FAIR CBF map for each mouse was fused to the T2w anatomical images in OsiriX (http://www.osirix-viewer.com). Using the T2w images as anatomical reference, fixed-size oval regions-of-interest (ROI) were drawn in the SVZs on the FAIR CBF maps to measure the mean image intensities in the SVZs. The mean image intensities of the ventricles were used as the background. 3D images were generated using Amira (VSG, Burlington, MA) after segmentation for the brain on T2w images and fusing with the FAIR CBF maps.

### Volumetric 3D assays

To measure the volume of blood vessels, 100µm-thick sections were imaged under a Zeiss LSM 510 inverted confocal microscope in order to create z-stacks. Those were analyzed using the Volocity 3D Image analysis software (Perkin Elmer) software to create three-dimensional angiograms and calculate the total volume.

### Imaging

Imaging was performed using a Zeiss LSM 510 Inverted Confocal microscope, a Nikon Eclipse Ti microscope and the Operetta High Content Imaging System (Perkin Elmer).

### Quantification of immunostaining

In all experiments, quantification was performed blindly: each experiment was assessed a code which was revealed after analysis. For the *in vivo* experiments, n represents the number of mice used for the analysis. For each mouse, 8-10 different sections comprising the same area of the SVZ were analyzed. Quantification of all figures was performed per field of view except for Olig2, which was measured only in the SVZ as marked by GFAP⁺ cells. Sox2 quantification in **Fig. 4C** was performed using a rectangular shape that was copied in all the images. Only cells inside the shape were counted. In endothelial cell proliferation assays, images of the whole well were acquired using the Operetta high content automated imaging microscope (Perkin Elmer) and were analyzed using Columbus Image Analysis Software (Perkin Elmer). Each *in vitro* experiment was repeated at least 3 times.

### Statistical analysis

Statistical analyses were performed using Graph Pad Prism software, with Student's t-test assuming two-tailed distribution and equal or unequal variances depending on the experiment. Statistical significance was assigned for p<0.05; results are shown as standard error of the mean.

### REFERENCES

1. G. Kempermann, H. G. Kuhn, F. H. Gage, Proc Natl Acad Sci U S A 94, 10409 (Sep 16, 1997).
2. A. Gritti et al., J Neurosci 22, 437 (Jan 15, 2002).
3. K. Sawamoto et al., Science 311, 629 (Feb 3, 2006).
4. E. Kokovay et al., Cell Stem Cell 7, 163 (Aug 6).
5. Q. Shen et al., Cell Stem Cell 3, 289 (Sep 11, 2008).
6. M. Tavazoie et al., Cell Stem Cell 3, 279 (Sep 11, 2008).
7. E. L. Jackson et al., Neuron 51, 187 (Jul 20, 2006).
8. C. Ramirez-Castillejo et al., Nat Neurosci 9, 331 (Mar, 2006).
9. F. Doetsch, L. Petreanu, I. Caille, J. M. Garcia-Verdugo, A. Alvarez-Buylla, Neuron 36, 1021 (Dec 19, 2002).
10. T. Shingo et al., Science 299, 117 (Jan 3, 2003).
11. P. Wu et al., Neurobiol Aging 29, 1502 (Oct, 2008).
12. H. van Praag, G. Kempermann, F. H. Gage, Nat Neurosci 2, 266 (Mar, 1999).
13. J. S. Snyder, A. Soumier, M. Brewer, J. Pickel, H. A. Cameron, Nature 476, 458 (Aug 25).
14. H. G. Kuhn, H. Dickinson-Anson, F. H. Gage, J Neurosci 16, 2027 (Mar 15, 1996).
15. T. Seki, Y. Arai, Neuroreport 6, 2479 (Dec 15, 1995).
16. B. Hattiangady, M. S. Rao, A. K. Shetty, Aging Cell 7, 207 (Mar, 2008).
17. M. J. Reed, J. M. Edelberg, Sci Aging Knowledge Environ 2004, pe7 (Feb 18, 2004).
18. A. Rivard et al., Circulation 99, 111 (Jan 5-12, 1999).
19. D. R. Riddle, W. E. Sonntag, R. J. Lichtenwalner, Ageing Res Rev 2, 149 (Apr, 2003).
20. D. E. Wright, A. J. Wagers, A. P. Gulati, F. L. Johnson, I. L. Weissman, Science 294, 1933 (Nov 30, 2001).
21. B. A. Reynolds, S. Weiss, Science 255, 1707 (Mar 27, 1992).
22. L. Katsimpardi et al., Stem Cells 26, 1796 (Jul, 2008).
23. A. Carleton, L. T. Petreanu, R. Lansford, A. Alvarez-Buylla, P. M. Lledo, Nat Neurosci 6, 507 (May, 2003).
24. R. M. Witt, M. M. Galligan, J. R. Despinoy, R. Segal, J Vis Exp (2009).
25. Y. Lin et al., Nature 455, 1198 (Oct 30, 2008).
26. Q. Shen et al., Science 304, 1338 (May 28, 2004).
27. C. L. Grady et al., Neuroimage 8, 409 (Nov, 1998).
28. A. J. Wagers, R. I. Sherwood, J. L. Christensen, I. L. Weissman, Science 297, 2256 (Sep 27, 2002).
29. S. G. Kim, Magn Reson Med 34, 293 (Sep, 1995).
30. Smyth G.K. Limma: Linear models for microarray data. In: Gentleman V., et al., editors.Bioinformatics and computational biology solutions using R and bioconductor R. Springer; New York: 2005. pp. 397-420.
31. S.A. Villeda et al., Nature 477, 90 (Sep 1, 2011).

## Claims

1. An agent or composition which increases the level of GDF11 polypeptide in a subject, thereby increasing neurogenesis in the subject, for use in treating or preventing a neurodegenerative disorder in a subject, wherein the agent or composition comprises a Growth and Differentiation Factor 11 (GDF11) polypeptide of SEQ ID NO: 3.

2. The agent or composition for use of claim 1, wherein increasing neurogenesis is associated with increased neural cell proliferation, increased neural cell differentiation, increased number or proliferation rate of neural stem cells, increased number or proliferation rate of neural progenitor cells, increased number or proliferation rate of neural precursor cells, increased expression of at least one synaptic plasticity gene, at least one neuroprotective gene, or at least one neuronal specification gene, or increased number of new neurons.

3. The agent or composition for use of claim 2, wherein the at least one synaptic plasticity gene, the at least one neuroprotective gene, and/or the at least one neuronal specification gene is selected from the group consisting of neuronal PAS domain protein 4 (Npas4), dual specificity phosphatase 1 (Dusp1), heat shock 70kDa protein 8 (Hspa8), BTG family, member 2 (Btg2), activity-regulated cytoskeleton-associated protein (Arc), nuclear receptor subfamily 4, group A, member 1 (Nr4a1), cysteine-rich, angiogenic inducer, 61 (Cyr61), FBJ murine osteosarcoma viral oncogene homolog (Fos), jun B proto-oncogene (Junb), Kruppel-like factor 10 (Klf10), nuclear receptor subfamily 4, group A, member 2 (Nr4a2), claudin 1 (Cldn1), early growth response 2 (Egr2), crystalline, mu (Crym), early growth response 1 (Egr1), and polio-like kinase 2 (Plk2).

4. The agent or composition for use of claim 1, wherein neurogenesis is increased in the subventricular zone of the lateral ventricles.

5. The agent or composition for use of claim 1, wherein neurogenesis is increased in the olfactory bulb.

6. The agent or composition for use of claim 5, wherein increased neurogenesis results in improved olfactory behavior.

7. The agent or composition for use of claim 1, wherein the subject has been diagnosed with a neurodegenerative disorder due to aging.

8. The agent or composition for use of claim 1, wherein the level of GDF11 polypeptide is increased in at least one of the subject's systemic circulation, cerebral vasculature, cerebral tissue, cerebrospinal fluid, lateral ventricles, neurovasculature, and subventricular zone neurovascular niche.

9. The agent or composition for use of claim 1, wherein the agent or composition comprises GDF11 polypeptide homodimers comprising polypeptides of the amino acid sequence of SEQ ID NO: 3.

10. A pharmaceutical composition comprising a GDF11 polypeptide of SEQ ID NO: 3, which increases the level of GDF11 polypeptide in a subject, thereby increasing neurogenesis in the subject, and a pharmaceutically acceptable carrier, for use in treating or preventing a neurodegenerative disorder in the subject.

## Patentansprüche

1. Agens oder Zusammensetzung, welche das Level von GDF11-Polypeptid in einem Subjekt erhöht, wodurch Neurogenese in dem Subjekt erhöht wird, zur Verwendung bei der Behandlung oder der Vorbeugung einer neurodegenerativen Erkrankung in einem Subjekt, worin die Agens oder Zusammensetzung ein Wachstums- und Differenzierungs-Faktor-11-(GDF11) Polypeptid von SEQ ID NO: 3 umfasst.

2. Agens oder Zusammensetzung zur Verwendung nach Anspruch 1, worin eine Erhöhung der Neurogenese mit erhöhter neuronaler Zellproliferation, erhöhter neuronaler Zelldifferenzierung, erhöhter Anzahl oder Proliferationsrate neuronaler Stammzellen, erhöhter Anzahl oder Proliferationsrate neuronaler Vorläuferzellen, erhöhter Anzahl oder Proliferationsrate von Neuroblasten, erhöhter Expression von mindestens einem synaptischen Plastizitätsgen, mindestens einem neuroprotektiven Gen, oder mindestens einem neuronalen Spezifikationsgen, oder einer erhöhten Anzahl junger Neurone verbunden ist.

3. Agens oder Zusammensetzung zur Verwendung nach Anspruch 2, worin das mindestens eine synaptische Plastizitätsgen, das mindestens eine neuroprotektive Gen, und/oder das mindestens eine neuronale Spezifikationsgen ausgewählt ist aus der Gruppe bestehend aus dem neuronalem PAS-Domänenprotein-4 (Npas4), der Dualspezifität-Phosphatase-1 (Dusp1), dem 70kDa-Hitzeschockprotein-8 (Hspa8), dem Mitglied-2 der BTG-Familie (Btg2), dem aktivitätsreguliertem Zytoskelett-assoziiertem Protein (Arc), dem Kernrezeptor-1 der Subfamilie-4, Gruppe-A (Nr4al), dem Cystein-reichen Angiogenese-Induktor-61 (Cyr61), dem murinen viralen FBJ-Osteosarkom-Onkogenhomolog (Fos), dem Jun-b-Protoonkogen (junb), dem Krüppel-ähnlichen Faktor-10 (Klf10), dem Kernrezeptor-2 der Subfamilie-4, Gruppe-A (Nr4a2), Claudin-1 (Cldnl), dem frühen Wachstumsantwort-Protein-2 (Egr2), Mu-Crystallin (Crym), dem frühen Wachstumsantwort-Protein-1 (Egr1), und der Polio-ähnlichen Kinase-2 (Plk2).

4. Agens oder Zusammensetzung zur Verwendung nach Anspruch 1, wobei Neurogenese in der subventrikulären Zone der Seitenventrikel erhöht wird.

5. Agens oder Zusammensetzung zur Verwendung nach Anspruch 1, wobei Neurogenese in dem olfaktorischen Bulbus erhöht wird.

6. Agens oder Zusammensetzung zur Verwendung nach Anspruch 5, wobei eine erhöhte Neurogenese zu verbessertem olfaktorischem Verhalten führt.

7. Agens oder Zusammensetzung zur Verwendung nach Anspruch 1, wobei das Subjekt mit einer neurodegenerativen Erkrankung aufgrund von Alterung diagnostiziert wurde.

8. Agens oder Zusammensetzung zur Verwendung nach Anspruch 1, wobei das Level von GDF11-Polypeptid in mindestens einem von dem systemischen Kreislauf, der Blutversorgung des Gehirns, dem Hirngewebe, der Cerebrospinalflüssigkeit, den Seitenventrikeln, der Neurovaskulatur, und der neurovaskularen Nische der subventrikulären Zone des Subjektes erhöht ist.

9. Agens oder Zusammensetzung zur Verwendung nach Anspruch 1, worin das Agens oder die Zusammensetzung GDF11-Polypeptid-Homodimere umfasst, die Polypeptide der Aminosäuresequenz von SEQ ID NO: 3 umfassen.

10. Pharmazeutische Zusammensetzung, welche ein GDF11-Polypeptid von SEQ ID 3, das das Level von GDF11-Polypeptid in einem Subjekt erhöht, wodurch Neurogenese in dem Subjekt erhöht wird, und einen pharmazeutisch annehmbaren Träger umfasst, zur Verwendung bei der Behandlung oder der Vorbeugung einer neurodegenerativen Erkrankung in dem Subjekt.

## Revendications

1. Agent ou composition qui augmente le taux de polypeptide GDF11 chez un sujet, ce qui augmente la neurogenèse chez le sujet, pour une utilisation dans le traitement ou la prévention d'un trouble neurodégénératif chez un sujet, dans lequel l'agent ou la composition comprend un polypeptide de facteur 11 de croissance et de différentiation (GDF11) de SEQ ID NO : 3.

2. Agent ou composition pour une utilisation selon la revendication 1, dans lequel l'augmentation de la neurogenèse est associée à une prolifération accrue des cellules neuronales, à une différenciation accrue des cellules neuronales, à une augmentation du nombre ou du taux de prolifération de cellules souches neuronales, à une augmentation du nombre ou du taux de prolifération de cellules progénitrices neuronales, à une augmentation du nombre ou du taux de prolifération de cellules précurseurs neuronales, à une expression accrue d'au moins un gène de plasticité synaptique, d'au moins un gène neuroprotecteur ou d'au moins un gène de spécification neuronale, ou à une augmentation du nombre de nouveaux neurones.

3. Agent ou composition pour une utilisation selon la revendication 2, dans lequel l'au moins un gène de plasticité synaptique, l'au moins un gène neuroprotecteur et/ou l'au moins un gène de spécification neuronale sont sélectionnés dans le groupe consistant en la protéine 4 de domaine PAS neuronal (Npas4), la phosphatase 1 à double spécificité (Dusp1), la protéine 8 de choc thermique de 70 kDa (Hspa8), le membre 2 de la famille BTG (Btg2), la protéine associée au cytosquelette à activité régulée (Arc), le membre 1 du groupe A de la sous-famille 4 de récepteurs nucléaires (Nr4a1), l'inducteur angiogénique 61 riche en cystéine (Cyr61), l'homologue d'oncogène viral d'ostéosarcome murin FBJ (Fos), le proto-oncogène jun B (Junb), le facteur 10 de type Krüppel (Klf10), le membre 2 du groupe A de la sous-famille 4 de récepteurs nucléaires (Nr4a2), la claudine 1 (Cldn1), la réponse 2 de croissance précoce (Egr2), la mu cristalline (Crym), la réponse 1 de croissance précoce (Egr1) et la polio like kinase 2 (Plk2).

4. Agent ou composition pour une utilisation selon la revendication 1, dans lequel la neurogenèse est augmentée dans la zone sous-ventriculaire des ventricules latéraux.

5. Agent ou composition pour une utilisation selon la revendication 1, dans lequel la neurogenèse est augmentée dans le bulbe olfactif.

6. Agent ou composition pour une utilisation selon la revendication 5, dans lequel une neurogenèse accrue entraîne un comportement olfactif amélioré.

7. Agent ou composition pour une utilisation selon la revendication 1, dans lequel le sujet a été diagnostiqué avec un trouble neurodégénératif dû au vieillissement.

8. Agent ou composition pour une utilisation selon la revendication 1, dans lequel le taux de polypeptide GDF11 est augmenté dans au moins l'un parmi la circulation systémique, le système vasculaire cérébral, le tissu cérébral, le liquide céphalo-rachidien, les ventricules latéraux, la neurovascularisation et la niche neurovasculaire de la zone sous-ventriculaire du sujet.

9. Agent ou composition pour une utilisation selon la revendication 1, dans lequel l'agent ou la composition comprend des homodimères de polypeptide GDF11 comprenant des polypeptides de la séquence d'acides aminés de SEQ ID NO : 3.

10. Composition pharmaceutique comprenant un polypeptide GDF11 de SEQ ID NO : 3, qui augmente le taux de polypeptide GDF11 chez un sujet, ce qui augmente la neurogenèse chez le sujet, et un support pharmaceutiquement acceptable, pour une utilisation dans le traitement ou la prévention d'un trouble neurodégénératif chez le sujet.
